(19) Europäisches Patentamt
European Patent Office
Office européen des brevets

(11) **EP 1 163 239 B1**

(12) # EUROPÄISCHE PATENTSCHRIFT

(45) Veröffentlichungstag und Bekanntmachung des Hinweises auf die Patenterteilung:
**28.05.2003 Patentblatt 2003/22**

(21) Anmeldenummer: **00922505.3**

(22) Anmeldetag: **08.03.2000**

(51) Int Cl.[7]: **C07D 401/14**, C07D 471/04, G01N 33/58, A61K 31/513, A61K 31/4178, A61K 31/551, A61K 31/4196, A61P 9/00, A61P 3/10

(86) Internationale Anmeldenummer:
**PCT/EP00/02004**

(87) Internationale Veröffentlichungsnummer:
**WO 00/055154 (21.09.2000 Gazette 2000/38)**

(54) **ABGEWANDELTE AMINOSÄUREAMIDE ALS CGRP ANTAGONISTEN**

MODIFIED AMINO-ACID AMIDES AS CGRP ANTAGONISTS

AMIDES D'AMINOACIDES MODIFIES COMME ANTAGONISTES DE LA CGRP

(84) Benannte Vertragsstaaten:
**AT BE CH CY DE DK ES FI FR GB GR IE IT LI LU MC NL PT SE**

(30) Priorität: **12.03.1999 DE 19911039**

(43) Veröffentlichungstag der Anmeldung:
**19.12.2001 Patentblatt 2001/51**

(73) Patentinhaber: **Boehringer Ingelheim Pharma GmbH & Co.KG**
**55218 Ingelheim am Rhein (DE)**

(72) Erfinder:
- **EBERLEIN, Wolfgang**
  **D-88400 Biberach (DE)**
- **RUDOLF, Klaus**
  **D-88447 Warthausen (DE)**
- **ENGEL, Wolfhard**
  **D-88400 Biberach (DE)**
- **DOODS, Henri**
  **D-88447 Warthausen (DE)**
- **HALLERMAYER, Gerhard**
  **D-88437 Maselheim-Sulmingen (DE)**

(74) Vertreter: **Laudien, Dieter, Dr.**
**Boehringer Ingelheim GmbH,**
**CD Patents**
**55216 Ingelheim am Rhein (DE)**

(56) Entgegenhaltungen:
**WO-A-00/18764** **WO-A-98/11128**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 163 239 B1

## Beschreibung

Gegenstand der vorliegenden Erfindung sind abgewandelte Aminosäureamide der allgemeinen Formel

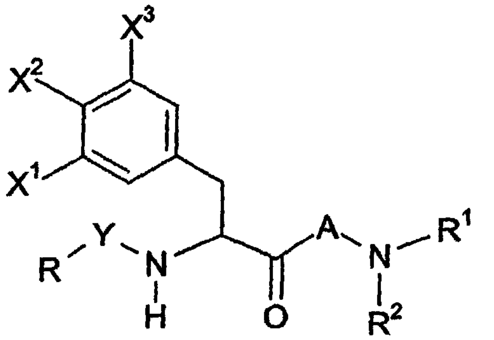

(I),

deren Tautomere, deren Diastereomere, deren Enantiomere, deren Gemische und deren Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In der obigen allgemeinen Formel I bedeuten

R die 1-Piperidinylgruppe, die in 4-Stellung durch einen über ein Stickstoffatom gebundenen, einfach oder zweifach ungesättigten 5- bis 7-gliedrigen Aza-, Diaza- oder Triaza-Heterocyclus, der ein oder zwei mit einem Stickstoffatom verknüpfte Carbonylgruppen enthält, substituiert ist,

wobei die vorstehend erwähnten Heterocyclen an einem Kohlenstoffatom durch eine Phenylgruppe substituiert sein können,

eine olefinische Doppelbindung eines der vorstehend erwähnten ungesättigten Heterocyclen mit einem Benzol-, Pyridin- oder Chinolin-Ring kondensiert sein kann oder zwei olefinische Doppelbindungen in einem der vorstehend erwähnten ungesättigten Heterocyclen benzokondensiert sein können,

und wobei die vorstehend erwähnten kondensierten Heterocyclen im Kohlenstoffgerüst und/oder an den in diesen Gruppen enthaltenen Phenylgruppen durch Fluor-, Chlor- oder Bromatome, durch $C_{1-3}$-Alkyl-, Trifluormethyl-, $C_{1-3}$-Alkoxy-, Hydroxy-, Amino-, Nitro-, Phenyl-, Carboxy-, Methoxycarbonyl-, Ethoxycarbonyl-, Aminocarbonyl-, Methylaminocarbonyl-, Hydroxyethylaminocarbonyl-, (4-Morpholinyl)carbonyl-, (1-Piperidinyl)carbonyl- oder (4-Methyl-1-piperazinyl)carbonylgruppen mono-, di- oder trisubstituiert sein können,

wobei die Substituenten gleich oder verschieden sein können und eine Mehrfachsubstitution mit den drei letztgenannten Substituenten ausgeschlossen ist,

und wobei insbesondere die Monosubstitution und als Substituenten die $C_{1-3}$-Alkyl-, $C_{1-3}$-Alkoxy- und Phenylgruppe besonders bevorzugt sind,

Y die zweiwertigen Reste

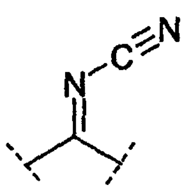

, $-SO_2-$ oder

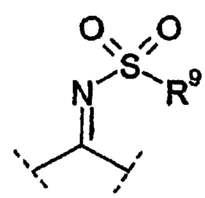

worin $R^9$ einen $C_{1-3}$-Alkylrest oder einen gegebenenfalls durch ein Fluor-, Chlor- oder Bromatom, eine Methyl- oder eine Methoxygruppe substituierten Phenylrest darstellt,

$X^1$, $X^2$ und $X^3$, die gleich oder verschieden sein können, das Wasserstoffatom, das Fluor-, Chlor- oder Bromatom, eine $C_{1-3}$-Alkyl-, $C_{1-3}$-Alkoxy-, Trifluormethyl-, Hydroxy-, Aminooder Acetylaminogruppe,

A eine Bindung oder den über die -CO-Gruppe mit der $NR^1R^2$-Gruppe der Formel I verknüpften zweiwertigen Rest

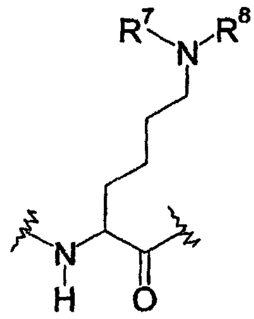

(II),

in dem

$R^7$ und $R^8$ unabhängig voneinander jeweils das Wasserstoffatom oder die Methylgruppe darstellen,

$R^1$ das Wasserstoffatom oder

eine in ω-Stellung gegebenenfalls durch eine Amino-, Methylamino-, Dimethylamino- oder 4- (1-Piperidinyl) -1-piperidinyl-Gruppe substituierte Alkylgruppe mit 1 bis 4 Kohlenstoffatomen, und

$R^2$ das Wasserstoffatom, die Methyl- oder Ethylgruppe oder

$R^1$ und $R^2$ zusammen mit dem eingeschlossenen Stickstoffatom einen Rest der allgemeinen Formel

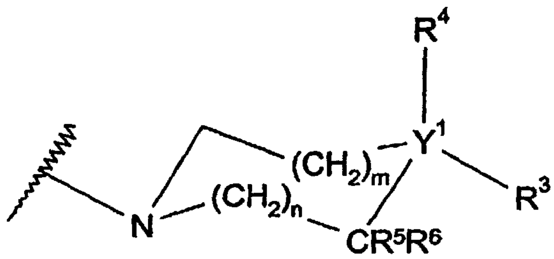

(III),

in der

$Y^1$ das Kohlenstoffatom oder, wenn $R^4$ ein freies Elektronenpaar darstellt, auch das Stickstoffatom,

m die Zahlen 0 oder 1,

n die Zahlen 1 oder 2,

$R^3$ das Wasserstoffatom,

eine Phenyl-, Pyridinyl- oder Diazinylgruppe, die jeweils im Kohlenstoffgerüst durch ein Fluor-, Chlor- oder Bromatom, durch eine Methyl- oder Methoxygruppe substituiert sein können,

eine 5- bis 7-gliedrige Azacycloalkylgruppe, eine 5- bis 7-gliedrige Oxaza- oder Diazacycloalkylgruppe oder eine 7-bis 9-gliedrige Azabicycloalkylgruppe,

wobei die vorstehend genannten mono- und bicyclischen Heterocyclen über ein Stickstoff- oder ein Kohlenstoffatom gebunden sind und

durch eine Alkylgruppe mit 1 bis 3 Kohlenstoffatomen, durch eine $C_{1-4}$-Alkanoyl-, Di-$C_{1-3}$-alkylamino- oder $C_{1-3}$-Alkylsulfonylgruppe substituiert sein können,

$R^4$ ein Wasserstoffatom,

einen Alkylrest mit 1 bis 3 Kohlenstoffatomen, wobei ein unverzweigter Alkylrest in ω-Stellung durch eine Phenyloder Pyridinylgruppe substituiert sein kann,

oder ein freies Elektronenpaar, wenn $Y^1$ ein Stickstoffatom darstellt, und

$R^5$ und $R^6$ jeweils ein Wasserstoffatom darstellen.

Beispielsweise kommen für $R^3$ die 1-Pyrrolidinyl-, 1-Piperidinyl-, 4-(Dimethylamino)-1-piperidinyl-, 4-Piperidinyl- oder 4-Morpholinylgruppe, wobei das Stickstoffatom der 4-Piperidinylgruppe durch eine Alkanoyl- oder Alkylgruppe mit jeweils 1 bis 4 Kohlenstoffatomen oder durch eine Methylsulfonylgruppe substituiert sein kann, die Hexahydro-1H-1-azepinyl-, 8-Methyl-8-azabicyclo[3,2,1]oct-3-yl-, 4-Alkyl-1-piperazinyl-, Hexahydro-4-alkyl-1H-1,4-diazepin-1-yl-, 1-Alkyl-4-piperidinylcarbonyl- oder 4-Alkyl-1-piperazinylcarbonylgruppe in Betracht.

Beispielsweise kommt für R die 4-(1,3-Dihydro-4-phenyl-2(2H)-oxoimidazol-1-yl)-1-piperidinyl-, 4-(1,3-Dihydro-2(2H)-oxobenzimidazol-1-yl)-1-piperidinyl-, 4-[2,4(1H,3H)-Dioxochinazolin-3-yl]-1-piperidinyl-, 4-(1,3-Dihydro-2(2H)-oxoimidazo[4,5-b]pyridin-3-yl-, 4-(3,4-Dihydro-2(1H)-oxochinazolin-3-yl)-1-piperidinyl-, 4-(2,3,4,5-Tetrahydro-2 (1H)-oxo-1,3-benzodiazepin-3-yl)-1-piperidinyl-, 4-(7-Methoxy-2,3,4,5-tetrahydro-2(1H)-oxo-1,3-benzodiazepin-3-yl) -1-piperidinyl-, 4-[2(1H)-Oxochinolin-3-yl]-1-piperidinyl-, 4-(2,4-Dihydro-5-phenyl-3(3H)-oxo-1,2,4-triazol-2-yl)-1-piperidinyl-, 4-(1,3-Dihydro-2(2H)-oxoimidazo[4,5-c]chinolin-3-yl)-1-piperidinyl- oder 4-(5,7-Dihydro-6-oxo-dibenzo[d,f] [1,3]diazepin-5-yl)-1-piperidinyl-Gruppe in Betracht.

Unter den in den vor- und nachstehenden Definitionen genannten Schutzresten sind die aus der Peptidchemie geläufigen Schutzgruppen zu verstehen, insbesondere

eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,

beispielsweise die Benzyloxycarbonyl-, 2-Nitro-benzyloxycarbonyl-, 4-Nitro-benzyloxycarbonyl-, 4-Methoxybenzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5 -Dimethoxy-α,α-dimethyl-benzyloxycarbonylgruppe,

eine Alkoxycarbonylgruppe mit insgesamt 1 bis 5 Kohlenstoffatomen im Alkylteil,

beispielsweise die Methoxycarbonyl -, Ethoxycarbonyl-, n-Propoxycarbonyl-, Isopropoxycarbonyl-, n-Butoxycarbonyl-, 1-Methylpropoxycarbonyl-, 2-Methylpropoxycarbonyl- oder tert.Butyloxycarbonylgruppe,

die Allyloxycarbonyl-, 2,2,2-Trichlorethoxycarbonyl- oder 9-Fluorenylmethoxycarbonyl-Gruppe oder

die Formyl-, Acetyl- oder Trifluoracetylgruppe.

Die vorliegende Erfindung betrifft Racemate, sofern die Verbindungen der allgemeinen Formel I nur ein Chiralitätselement besitzen. Die Anmeldung umfaßt jedoch auch die einzelnen diastereomeren Antipodenpaare oder deren Gemische, die dann vorliegen, wenn mehr als ein Chiralitätselement in den Verbindungen der allgemeinen Formel (I) vorhanden ist.

Bevorzugt werden die unter die allgemeine Formel I fallenden Verbindungen, die hinsichtlich der Aminosäure-Partialstruktur der Formel

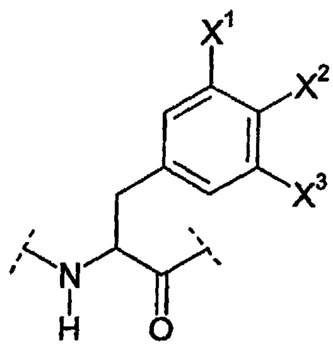

(IV)

D- bzw. (R)-konfiguriert und hinsichtlich der im Rest A gegebenenfalls vorhandenen Aminosäure-Partialstruktur der Formel II L- bzw. (S)-konfiguriert sind.

Die Verbindungen der allgemeinen Formel I weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Besonders bevorzugte Verbindungen der obigen allgemeinen Formel I sind diejenigen, in denen

R die 1-Piperidinylgruppe bedeutet, die in 4-Stellung durch

eine 1,3-Dihydro-4-phenyl-2(2H)-oxoimidazol-1-yl-, 1,3-Dihydro-2(2H)-oxobenzimidazol-1-yl-, 2,4(1H,3H)-Dioxochinazolin-3-yl-, 1,3-Dihydro-2(2H)-oxoimidazo[4,5-b]pyridin-3-yl-, 3,4-Dihydro-2(1H)-oxochinazolin-3-yl-, 2,3,4,5-Tetrahydro-2(1H)-oxo-1,3-benzodiazepin-3-yl-, 2(1H)-Oxochinolin-3-yl-, 2,4-Dihydro-5-phenyl-3(3H)-oxo-1,2,4-triazol-2-yl-, 1,3-Dihydro-2(2H)-oxoimidazo[4,5-c] chinolin-3-yl- oder 5,7-Dihydro-6-oxo-dibenzo[d,f] [1,3]diazepin-5-yl-Gruppe substituiert ist,

wobei die vorstehend erwähnten bicyclischen Heterocyclen im Kohlenstoffgerüst und/oder an den in diesen Gruppen enthaltenen Phenylgruppen durch Fluor-, Chlor- oder Bromatome, durch Methyl-, Trifluormethyl-, Methoxy-, Hydroxy-, Amino-, Nitro-, Phenyl-, Phenylmethyl-, Carboxy-, Methoxycarbonyl-, Ethoxycarbonyl-, Aminocarbonyl-, Methylaminocarbonyl-, Hydroxyethylaminocarbonyl-, (4-Morpholinyl)carbonyl-, (1-Piperidinyl)carbonyl- oder (4-Methyl-1-piperazinyl)carbonylgruppen mono-, di- oder trisubstituiert sein können,

wobei die Substituenten gleich oder verschieden sein können und eine Mehrfachsubstitution mit den drei letztgenannten Substituenten ausgeschlossen ist,

und wobei insbesondere die Monosubstitution und als Substituenten die Methyl-, Methoxy- und Phenylgruppe

besonders bevorzugt sind,
Y die zweiwertigen Reste

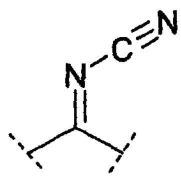 , $-SO_2-$ oder 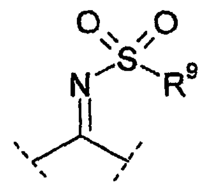

worin $R^9$ die Methylgruppe oder den Phenylrest darstellt,
$X^1$ das Fluor- Chlor- oder Bromatom oder die Methylgruppe,
$X^2$ das Fluor- Chlor- oder Bromatom, die Methyl-, Methoxy-, Hydroxy- oder Aminogruppe,
$X^3$ das Fluor- Chlor- oder Bromatom oder die Methylgruppe,
A eine Bindung oder den über die -CO-Gruppe mit der $NR^1R^2$-Gruppe der Formel I verknüpften zweiwertigen Rest

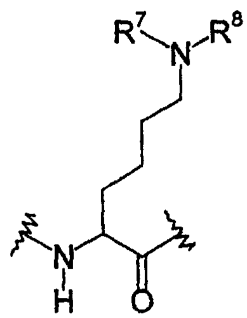 (II),

in dem
$R^7$ und $R^8$ Wasserstoffatome darstellen,
$R^1$ und $R^2$ zusammen mit dem eingeschlossenen Stickstoffatom einen Rest der allgemeinen Formel

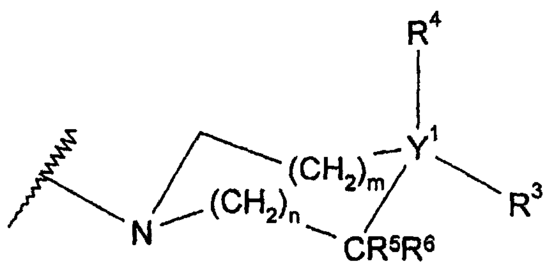 (III),

in der
$Y^1$ das Kohlenstoffatom oder, wenn $R^4$ ein freies Elektronenpaar darstellt, auch das Stickstoffatom,
m die Zahl 1,
n die Zahl 1,
$R^3$ eine Phenyl- oder Pyridinylgruppe, die jeweils im Kohlenstoffgerüst durch ein Fluor-, Chlor- oder Bromatom, durch eine Methyl- oder Methoxygruppe substituiert sein können,
eine 1-Pyrrolidinyl-, 1-Piperidinyl-, 4-(Dimethylamino)-1-piperidinyl-, 4-Piperidinyl- oder 4-Morpholinylgruppe, wobei das Stickstoffatom der 4-Piperidinylgruppe durch eine Alkylgruppe mit jeweils 1 bis 2 Kohlenstoffatomen substituiert sein kann, eine Hexahydro-1H-1-azepinyl-, 4-Methyl-1-piperazinyl- oder 4-Ethyl-1-piperazinylgruppe,
$R^4$ ein Wasserstoffatom, einen Alkylrest mit 1 oder 2 Kohlenstoffatomen oder ein freies Elektronenpaar, wenn $Y^1$ ein Stickstoffatom darstellt, und

$R^5$ und $R^6$ jeweils ein Wasserstoffatom darstellen,
bedeuten,
deren Tautomere, deren Diastereomere, deren Enantiomere, deren Gemische und deren Salze.

Als besonders bevorzugte Verbindungen seien beispielsweise folgende genannt:

(1) 1-[4-Amino-3,5-dibrom-N-[[4-(3,4-dihydro-2(1H)-oxochinazolin-3-yl)-1-piperidinyl]methylsulfonyliminomethyl]-D-phenylalanyl]-4-(1-piperidinyl)-piperidin,

(2) 1-[4-Amino-3,5-dibrom-N-[[4-(3,4-dihydro-2(1H)-oxochinazolin-3-yl)-1-piperidinyl]cyaniminomethyl]-D-phenylalanyl]-4-(1-piperidinyl)-piperidin,

(3) 1-[4-Amino-3,5-dibrom-N-[[4-(3,4-dihydro-2(1H)-oxochinazolin-3-yl)-1-piperidinyllphenylsulfonyliminomethyl]-D-phenylalanyl]-4-(1-piperidinyl)-piperidin,

(4) 1-[3,5-Dibrom-N-[[4-(3,4-dihydro-2(1H)-oxochinazolin-3-yl)-1-piperidinyl]cyaniminomethyl]-D-tyrosyl]-4-(1-piperidinyl)-piperidin,

(5) 1-[$N^2$-[3,5-Dibrom-N-[[4-(3,4-dihydro-2(1H)-oxochinazolin-3-yl)-1-piperidinyl]methylsulfonyliminomethyl]-D-tyrosyl]-L-lysyl]-4-(4-pyridinyl)-piperazin,

(6) 1- [$N^2$-[3,5-Dibrom-N-[[4-(3,4-dihydro-2(1H)-oxochinazolin-3-yl)-1-piperidinyl]phenylsulfonyliminomethyl]-D-tyrosyl]-L-lysyl]-4-(4-pyridinyl)-piperazin,

(7) 1- [4-Amino-3,5-dibrom-N-[[4-(3,4-dihydro-2(1H)-oxochinazolin-3-yl)-1-piperidinyl]cyaniminomethyl]-D-phenylalanyl]-4-(1-methyl-4-piperidinyl)-piperidin,

(8) 1-[4-Brom-N-[[4-(3,4-dihydro-2(1H) oxochinazolin-3-yl)1-piperidinyl]cyaniminomethyl]-3,5-dimethyl-D,L-phenylalanyl]-4- (1-piperidinyl)-piperidin,

(9) 1-[3,5-Dibrom-N-[[4-(3,4-dihydro-2(1H)-oxochinazolin-3-yl)-1-piperidinyl]cyaniminomethyl]-D-tyrosyl]-4-(4-pyridinyl)-piperazin,

(10) 1-[4-Amino-3,5-dibrom-N-[[4-(3,4-dihydro-2(1H)-oxochinazolin-3-yl)-1-piperidinyl]cyaniminomethyl]-D-phenylalanyl]-4-(4-pyridinyl)-piperazin,

(11) 1-[3,5-Dibrom-N-[[4-(3,4-dihydro-2 (1H)-oxochinazolin-3-yl)-1-piperidinyl]cyaniminomethyl]-D-tyrosyl]-4-(1-methyl-4-piperidinyl)-piperidin,

(12) 1-[3,5-Dibrom-N-[[4-[3,4-dihydro-2(1H)-oxochinazolin-3-yl]-1-piperidinyl]cyaniminomethyl]-D-tyrosyl]-4-(4-methyl-1-piperazinyl)piperidin,

(13) 1-[4-Brom-N-[[4-(3,4-dihydro-2(1H)-oxochinazolin-3-yl)-1-piperidinyl]cyaniminomethyl]-3,5-dimethyl-D,L-phenylalanyl]-4-(1-methyl-4-piperidinyl)-piperidin,

(14) 1-[4-Amino-3,5-dibrom-N-[[4-[1,3-dihydro-4-phenyl-2(2H)-oxoimidazol-1-yl]-1-piperidinyl]cyaniminomethyl]-D-phenylalanyl]-4-(4-methyl-1-piperazinyl)-piperidin,

(15) 1-[4-Amino-3,5-dibrom-N-[[4-(2,3,4,5-tetrahydro-2(1H)-oxo-1,3-benzodiazepin-3-yl)-1-piperidinyl]cyaniminomethyl]-D-phenylalanyl]-4-(4-methyl-1-piperazinyl)-piperidin,

(16) 1-[4-Amino-3,5-dibrom-N-[[4-(2,4-dihydro-5-phenyl-3(3H)-oxo-1,2,4-triazol-2-yl)-1-piperidinyl]cyaniminomethyl]-D-phenylalanyl]-4-(1-piperidinyl)-piperidin,

(17) 1-[4-Amino-3,5-dibrom-N-[[4-(2,3,4,5-tetrahydro-2(1H)-oxo-1,3-benzodiazepin-3-yl)-1-piperidinyl]cyaniminomethyl]-D-phenylalanyl]-4-(1-piperidinyl)-piperidin,

(18) 1-[3,5-Dibrom-N-[[4-(2,4-dihydro-5-phenyl-3(3H)-oxo-1,2,4-triazol-2-yl)-1-piperidinyl]cyaniminomethyl]-D-tyrosyl]-4-(1-piperidinyl)-piperidin,

(19) 1-[3,5-Dibrom-N-[[4-(2,3,4,5-tetrahydro-2(1H)-oxo-1,3-benzodiazepin-3-yl)-1-piperidinyl]cyaniminomethyl]-D-tyrosyl]-4-(1-piperidinyl)-piperidin,

(20) 1-[3,5-Dibrom-N-[[4-(2,4-dihydro-5-phenyl-3(3H)-oxo-1,2,4-triazol-2-yl)-1-piperidinyl]cyaniminomethyl]-D-tyrosyl]-4-(1-methyl-4-piperidinyl)-piperazin,

(21) 1-[3,5-Dibrom-N-[[4-(2,3,4,5-tetrahydro-2(1H)-oxo-1,3-benzodiazepin-3-yl)-1-piperidinyl]cyaniminomethyl]-D-tyrosyl]-4-(1-methyl-4-piperidinyl)-piperazin,

(22) 1- [4-Amino-3,5-dibrom-N-[[4-(2,4-dihydro-5-phenyl-3(3H)-oxo-1,2,4-triazol-2-yl)-1-piperidinyl]cyaniminomethyl]-D-phenylalanyl]-4-(1-methyl-4-piperidinyl)-piperazin,

(23) 1-[4-Amino-3,5-dibrom-N-[[4-(2,3,4,5-tetrahydro-2(1H)-oxo-1,3-benzodiazepin-3-yl)-1-piperidinyl]cyaniminomethyl)-D-phenylalanyl]-4-(1-methyl-4-piperidinyl)-piperazin,

(24) 1-[3,5-Dibrom-N-[[4-(3,4-dihydro-2(1H)-oxochinazolin-3-yl)-1-piperidinyl]cyaniminomethyl]-4-methyl-D,L-phenylalanyl]-4-(1-methyl-4-piperidinyl)-piperidin,

(25) 1-[3,5-Dibrom-N-[[4-(3,4-dihydro-2(1H)-oxochinazolin-3-yl)-1-piperidinyl]cyaniminomethyl]-4-methyl-D,L-phenylalanyl]-4-(1-piperidinyl)-piperidin,

(26) 1-[3,5-Dibrom-N-[[4-(3,4-dihydro-2(1H)-oxochinazolin-3-yl)-1-piperidinyl]cyaniminomethyl]-4-methyl-D,L-phenylalanyl]-4-(4-pyridinyl)-piperazin,

(27) 1-[4-Amino-3,5-dibrom-N-[[4-[1,3-dihydro-2(2H)-oxoimidazo[4,5-c]chinolin-3-yl]-1-piperidinyl]cyaniminomethyl]-D-phenylalanyl]-4-(1-piperidinyl)piperidin,

(28) 1-[4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(1-piperidinyl)-piperidin,

(29) 1-[4-Amino-3,5-dibrom-N-[[4-(5,7-dihydro-6-oxodibenzo-[d,f] [1,3]diazepin-5-yl)-1-piperidinyl]cyaniminomethyl]-D-phenylalanyl] -4-(1-piperidinyl)-piperidin,

(30) 1-[4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(1-methyl-4-piperidinyl)-piperazin,

(31) 1-[4-Amino-3,5-dibrom-N-[[4- [1,3-dihydro-2(2H)-oxoimidazo[4,5-c]chinolin-3-yl]-1-piperidinyl]cyaniminomethyl]-D-phenylalanyl]-4-(1-methyl-4-piperidinyl)piperazin,

(32) 1-[4-Amino-3,5-dibrom-N-[[4-(2,3,4,5-tetrahydro-2(1H)-oxo-1,3-benzodiazepin-3-yl)-1-piperidinyl]sulfonyl]-D-phenylalanyl] -4-(1-methyl-4-piperidinyl)-piperazin,

(33) 1-[3,5-Dibrom-N-[[4-(7-methoxy-2,3,4,5-tetrahydro-2(1H)-oxo-1,3-benzodiazepin-3-yl)-1-piperidinyl]cyaniminomethyl]-D-tyrosyl]-4-(1-piperidinyl)-piperidin,

(34) 1-[3,5-Dibrom-N-[[4-(7-methoxy-2,3,4,5-tetrahydro-2(1H)-oxo-1,3-benzodiazepin-3-yl)-1-piperidinyl]cyaniminomethyl]-D-tyrosyl]-4-(1-methyl-4-piperidinyl)-piperazin,

(35) 1-[3,5-Dibrom-N-[[4-[1,3-dihydro-2(2H)-oxoimidazo[4,5-c]-chinolin-3-yl]-1-piperidinyl]cyaniminomethyl]-D-tyrosyl]-4-(1-piperidinyl)piperidin,

(36) 1-[3,5-Dibrom-N-[[4-[1,3-dihydro-2(2H)-oxoimidazo[4,5-c]-chinolin-3-yl]-1-piperidinyl]cyaniminoniethyl]-D-tyrosyl]-4-(1-methyl-4-piperidinyl)piperazin,

(37) 1-[4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(4-methyl-1-piperazinyl)-piperidin und

(38) 1-[4-Amino-3,5-dibrom-N-[[4-[1,3-dihydro-2(2H)-oxoimidazo[4,5-c]chinolin-3-yl]-1-piperidinyl]cyaniminomethyl] -D-phenylalanyl]-4-(4-methyl-1-piperazinyl)piperidin

und deren Salze.

Die Verbindungen der allgemeinen Formel I werden nach prinzipiell bekannten Methoden hergestellt, wobei auch aus der Peptidchemie (siehe z. B. Houben-Weyl, Methoden der Organischen Chemie, Bd. 15/2) abgeleitete Verfahren angewandt werden. Als Aminoschutzgruppen können die in Houben-Weyl, Methoden der Organischen Chemie, Bd. 15/1, beschriebenen verwendet werden, wobei Urethanschutzgruppen, wie z. B. die Fluorenylmethoxycarbonyl-, Phenylmethoxycarbonyl- oder tert.-Butyloxycarbonylgruppe bevorzugt werden. Eventuell im Rest A der Verbindungen der allgemeinen Formel I oder in deren Vorstufen vorhandene funktionelle Gruppen werden zur Verhinderung von Nebenreaktionen durch geeignete Schutzgruppen (siehe z. B.: G.B. Fields et al., Int. J. Peptide Protein Res. 35, 161 (1990); T.W. Greene, Protective Groups in Organic Synthesis) zusätzlich geschützt. Als derartige seitenkettengeschützte Aminosäuren seien besonders Lys(Boc), Lys(Cl-Z) und Lys(Teoc) erwähnt, die, eventuell in Form von Derivaten, in der Regel käuflich sind.

Statt seitenkettenständige Aminogruppen zu schützen, können auch Präcursor-Funktionen tragende, in der Seitenkette insbesondere durch Nitro oder Cyan substituierte Aminosäuren bzw. deren Derivate eingesetzt werden, beispielsweise 5-Cyannorvalin. .

Eventuelle in den Seitenketten von a-Aminosäurepartialstrukturen vorhandene Schutzgruppen werden nach Aufbau des N- und C-terminal substituierten Aminosäurederivats abschließend mit geeigneten, im Prinzip gleichfalls literaturbekannten Reagenzien abgespalten, z. B. Arylmethoxycarbonylschutzgruppen hydrogenolytisch, beispielsweise mit Wasserstoff in Gegenwart von Palladiummohr und unter Verwendung von Eisessig als Lösemittel. .

In der Seitenkette der a-Aminosäure gegebenenfalls vorhandene Präcursor-Funktionen können gleichfalls abschließend durch Hydrogenolyse in die gewünschten Aminofunktionen übergeführt werden; Nitroalkylgruppen ergeben dabei unter dem Chemiker geläufigen Bedingungen Aminoalkylgruppen, die Cyangruppe geht in die Aminomethyl-Gruppe über.

Die folgenden Verfahren sind zur Herstellung der erfindungsgemäßen Verbindungen der allgemeinen Formel I besonders geeignet:

a) Zur Herstellung von Verbindungen der allgemeinen Formel I, in der Y einen der zweiwertigen Iminomethylreste

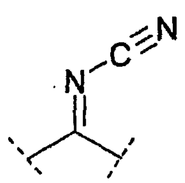

oder

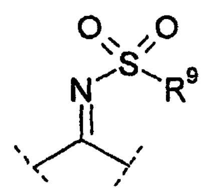

bedeutet, wobei $R^9$ wie eingangs angegeben definiert ist:
Umsetzung von Verbindungen der allgemeinen Formel

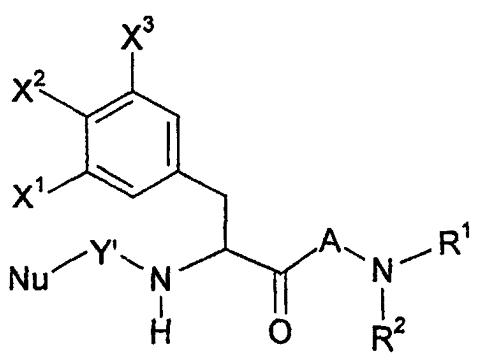

(V) ,

in der A, $R^1$, $R^2$, $X^1$, $X^2$ und $X^3$ wie eingangs definiert sind, Y' einen der beiden oben angegebenen Iminomethylreste darstellt und Nu eine Austrittsgruppe ist, beispielsweise eine Alkoxy-, Aryloxy-, Alkylthio-, Alkylsulfinyl- oder Alkylsulfonylgruppe mit jeweils bis zu 10 Kohlenstoffatomen, z. B. die Methoxy-, Ethoxy-, Phenyloxy-, Methylthio-, Ethylthio-, Methylsulfinyl-, Ethylsulfinyl-, Propylsulfinyl-, Isopropylsulfinyl-, Methylsulfonyl- oder Ethylsulfonylgruppe, das Chlor-

oder Bromatom, die $SO_2H$-, $SO_3H$- oder $OPOCl_2$-Gruppe, vorzugsweise jedoch die Phenoxygruppe, mit sekundären Aminen der allgemeinen Formel

R-H (VI),

in der R wie eingangs definiert ist.

Die Umsetzungen werden in Analogie zu literaturbekannten Verfahren ( siehe G. B. L. Smith, J. Amer. Chem. Soc. 51, 476 [1929]; B. Rathke, Chem. Ber. 17, 297 [1884]; R. Phillips und H. T. Clarke,. J. Amer. Chem. Soc. 45, 1755 [1923]; S. J. Angyal und W. K. Warburton, J. Amer. Chem. Soc. 73, 2492 [1951]; H. Lecher und F. Graf, Chem. Ber. 56, 1326 [1923]; J. Wityak, S. J. Gould, S. J. Hein und D. A. Keszler, J. Org. Chem. 52, 2179 [1987]; T. Teraji, Y. Nakai, G. J. Durant, WO-A-81/00109, Chem. Abstr. 94, 192336z [1981]; C. A. Maryanoff, R. C. Stanzione, J. N. Plampin und J. E. Mills, J. Org. Chem. 51, 1882 - 1884 [1986]; A. E. Miller und J. J. Bischoff, Synthesis 1986, 777; R. A. B. Bannard, A. A. Casselman, W. F. Cockburn und G. M. Brown, Can. J. Chem. 36, 1541 [1958]; Aktieselskabet Grea, Kopenhagen, DE2826452-C2; K. Kim, Y. T. Lin und H. S. Mosher, Tetrah. Letters 29, 3183-3186 [1988]; H. B. Arzeno et al., Synth. Commun. 20, 3433-3437 [1990]; H. Bredereck und K. Bredereck, Chem. Ber. 94, 2278 [1961]; H. Eilingsfeld, G. Neubauer, M. Seefelder und H. Weidinger, Chem. Ber. 97, 1232 [1964]; P. Pruszynski, Can. J. Chem. 65, 626 [1987]; D. F. Gavin, W. J. Schnabel, E. Kober und M. A. Robinson, J. Org. Chem. 32, 2511 [1967]; N. K. Hart, S. R. Johns, J. A. Lamberton und R. I. Willing, Aust. J. Chem. 23., 1679 [1970]; CIBA Ltd., Belgisches Patent 655403; Chem. Abstr. 64, 17481 [1966]; J. P. Greenstein, J. Org. Chem. 2, 480 [1937]; F. L. Scott und J. Reilly, J. Amer. Chem. Soc. 74, 4562 [1952]; W. R. Roush und A. E. Walts, J. Amer. Chem. Soc. 106, 721 [1984]; M. S. Bernatowicz, Y. Wu und G. R. Matsueda, J. Org. Chem. 57, 2497-2502 [1992]; H. Tsunematsu, T. Imamura und S. Makisumi, J. Biochem. 94, 123-128 [1983] ; R. Mohr, A. Buschauer und W. Schunack, Arch. Pharm. 321, 221-227 [1988]; K. Atwal, F. N. Ferrara und S. Z. Ahmed, Tetrah. Lett. 35., 8085-8088 [1994]; P. J. Garratt, C. J. Hobbs und R. Wrigglesworth, J. Org. Chem. 54, 1062-1069 [1989]; P. J. Garratt und S. N. Thorn, Tetrahedron 49, 6885-6898 [1993]) bei Temperaturen zwischen 0°C und +100°C, bevorzugt +40°C und +80°C, und unter Verwendung inerter Lösemittel, beispielsweise von Dichlormethan, Tetrahydrofuran, 1,4-Dioxan, Acetonitril, Dimethylformamid, 2-Pentanol, Dimethylacetamid, N-Methylpyrrolidon oder Gemischen davon und in der Regel in Gegenwart von Hilfsbasen, insbesondere von Alkalicarbonaten, wie Natrium- oder Kaliumcarbonat, oder von tertiären Aminen, bevorzugt N-Ethyldiisopropylamin oder Triethylamin, durchgeführt.

b) Zur Herstellung von Verbindungen der allgemeinen Formel I, in der Y den zweiwertigen Rest $-SO_2-$ bedeutet:

Umsetzung von Verbindungen der allgemeinen Formel

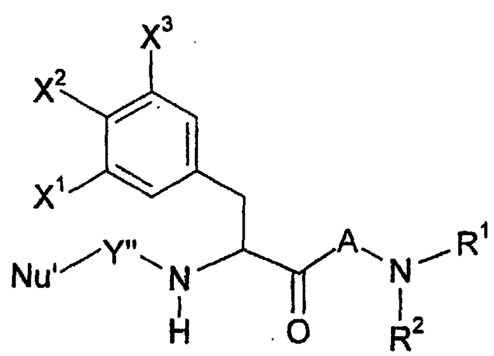

(VII),

in der A, $R^1$, $R^2$, $X^1$, $X^2$ und $X^3$ wie eingangs erwähnt definiert sind, Y' ' die $SO_2$-Gruppe bedeutet und Nu' eine Austrittsgruppe ist, beispielsweise ein Halogenatom, wie das Chlor-, Brom- oder Iodatom, eine Alkyl- oder Arylsulfonyloxygruppe oder eine Alkoxygruppe mit jeweils bis zu 10 Kohlenstoffatomen, z. B. die Methoxy- oder Ethoxygruppe, oder eine gegebenenfalls durch Chlor- oder Bromatome, durch Methyl-, Nitro- oder Hydroxygruppen mono-, di- oder trisubstituierte Phenoxy- oder Naphthoxygruppe, wobei die Substituenten gleich oder verschieden sein können, mit sekundären Aminen der allgemeinen Formel

R-H (VI),

in der R wie eingangs definiert ist, und, falls nötig, anschließende Abspaltung von Schutzgruppen oder Abwandlung von Präcursorfunktionen nach den vorstehend beschriebenen Verfahren.

Bedeutet in der allgemeinen Formel VII Nu' ein Halogenatom, eine Alkyl- oder Arylsulfonyloxygruppe, so wird die Umsetzung unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +100°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, , Kaliumhydroxid, Alkalicarbonate, z. B. Natriumcarbonat, Kaliumcarbonat oder Cäsiumcarbonat, Alkaliacetate, z. B. Natrium- oder Kaliumacetat, sowie tertiäre Amine, beispielsweise Pyridin, 2,4,6-Trimethylpyridin, Chinolin, Triethylamin, N-Ethyldiisopropylamin, N-Ethyldicyclohexylamin, 1,4-Diazabicyclo[2,2,2]octan oder 1,8-Diazabicyclo[5,4,0]undec-7-en, als Lösemittel beispielsweise Dichlormethan, Tetrahydrofuran, 1,4-Dioxan, Acetonitril, Dimethylformamid, Dimethylacetamid, N-Methylpyrrolidon oder Gemische davon in Betracht; werden als Hilfsbasen Alkali-oder Erdalkalihydroxide, Alkalicarbonate oder -acetate verwendet, kann dem Reaktionsgemisch auch Wasser als Cosolvens zugesetzt werden.

Als nucleofuge Gruppe Nu' in Verbindungen der allgemeinen Formel VII wird die 2-Hydroxyphenoxygruppe, als Lösemittel für die Umsetzung mit Aminen der allgemeinen Formel VI siedendes Dioxan bevorzugt.

Als Zwischenprodukte der Umsetzung sind die nicht isolierbaren Azasulfene der allgemeinen Formel

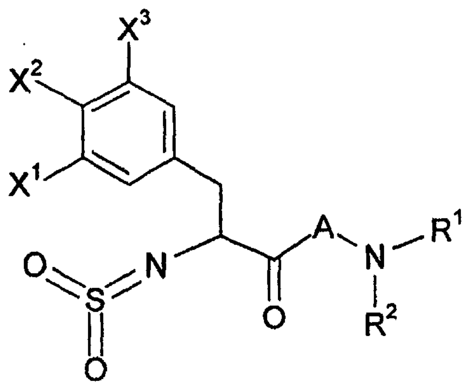

(VIII)

anzunehmen.

Die erfindungsgemäßen abgewandelten Aminosäuren der allgemeinen Formel I enthalten wenigstens ein Chiralitätszentrum. Ist auch der Rest A chiral, dann können die Verbindungen in Form zweier diastereomerer Antipodenpaare auftreten. Die Erfindung umfaßt die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel I fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diästereomeren, optisch aktiven Salze trennen, die bei Umsetzung mit einer optisch aktiven Säure, beispielsweise (+)-oder (-)-Weinsäure, (+)- oder (-)-Diacetylweinsäure, (+)- oder (-)-Monomethyltartrat oder (+)-Camphersulfonsäure, bzw. optisch aktiven Base, beispielsweise mit (R)-(+)-1-Phenylethylamin, (S)-(-) -1-Phenylethylamin oder (S)-Brucin, entstehen.

Nach einem üblichen Verfahren zur Isomerentrennung wird das Racemat einer Verbindung der allgemeinen Formel I mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösungsmitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, Matronlauge oder Kalilauge neutralisiert und dadurch die entsprechende freie Verbindung in der (+)- oder (-)-Form erhalten.

Jeweils nur das (R)-Enantiomer bzw. ein Gemisch zweier optisch aktiver, unter die allgemeine Formel I fallender diastereomerer Verbindungen wird auch dadurch erhalten, daß man die oben beschriebenen Synthesen mit jeweils einer geeigneten (R)-konfigurierten Reaktionskomponente durchführt.

Die zur Synthese der Verbindungen der allgemeinen Formel I erforderlichen Ausgangsmaterialien der allgemeinen Formeln V, VI und VII werden in Analogie zu literaturbekannten Verfahren hergestellt.

Ausgangsverbindungen der allgemeinen Formel V erhält man beispielsweise durch Umsetzung der bereits in der WO 98/11128 beschriebenen Phenylalaninderivate der allgemeinen Formel

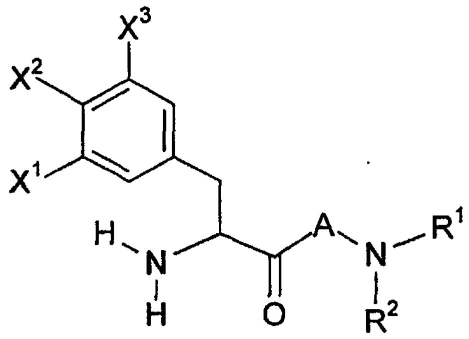
(IX),

worin A, $R^1$, $R^2$, $X^1$, $X^2$ und $X^3$ wie vorstehend definiert sind, mit Iminocarbonaten der allgemeinen Formel

Nu-Y′-Nu″ (X),

in der Nu und Y' wie vorstehend unter a) definiert sind und Nu", das von Nu verschieden oder auch gleich wie Nu sein kann, die gleichen Bedeutungen wie Nu annehmen kann. Die Reaktionen werden in Analogie zu Angaben von R. Mohr, A. Buschauer und W. Schunack, Arch. Pharm. 321, 221-227 [1988] bzw. A. Buschauer, Arch. Pharm. 320, 377-380 [1987] bzw. P. J. Garratt und S. N. Thorn, Tetrahedron 49 6885-6898 [1993] durchgeführt .

Verbindungen der allgemeinen Formel VI sind in der Regel bereits in der WO 98/11128 beschrieben worden. Die unter die allgemeine Formel VI fallende, noch nicht vorbeschriebene Verbindung

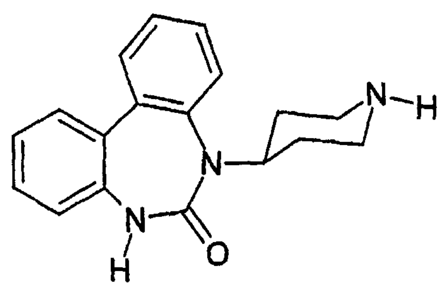
(XI)

läßt sich aus 2-Amino-2'-nitrobiphenyl durch reduktive Aminierung mit 1-(Phenylmethyl)-4-piperidon, anschließende Reduktion der Nitrogruppe, Cyclisierung unter Verwendung von N,N'-Carbonyldiimidazol und hydrogenolytische Entfernung der Benzylgruppe in Anlehnung an literaturbekannte Verfahren leicht herstellen.

Die als Ausgangsverbindungen benötigten Verbindungen der allgemeinen Formel VII lassen sich aus Phenylalaninderivaten der vorstehend definierten allgemeinen Formel IX durch Umsetzung mit Sulfaten der allgemeinen Formel

Nu' - Y" -Nu ''' (XII)

in der Nu' und Y" wie vorstehend unter b) definiert sind und Nu''', das von Nu' verschieden oder auch gleich wie Nu' sein kann, die gleichen Bedeutungen wie Nu' annehmen kann. Als Sulfat wird die cyclische Verbindung

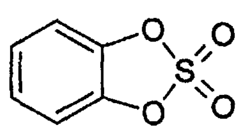
(XIII)

bevorzugt (siehe auch: G. E. DuBois und R. A. Stephenson, J. Org. Chem. 45, 5371-5373 [1980]).

Die erhaltenen Verbindungen der allgemeinen Formel I können, insbesondere für pharmazeutische Anwendungen, in ihre physiologisch verträglichen Salze mit anorganischen oder organischen Säuren, übergeführt werden. Als Säuren kommen hierfür beispielsweise Salzsäure, Bromwasserstoffsäure, Phosphorsäure, Salpetersäure, Schwefelsäure, Methansulfonsäure, p-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die so erhaltenen neuen Verbindungen der Formel I, falls diese eine saure Funktion, beispielsweise eine Carboxygruppe enthalten, gewünschtenfalls in ihre Additionssalze mit anorganischen oder organischen Basen, insbesondere für die pharmazeutische Anwendung geeigneten physiologisch verträglichen Additionssalze, überführen. Als Basen kommen hierbei beispielsweise Natriumhydroxid, Kaliumhydroxid, Ammoniak, Cyclohexylamin, Dicyclohexylamin, Äthanolamin, Diäthanolamin und Triäthanolamin in Betracht.

Die neuen Verbindungen der allgemeinen Formel I und deren physiologisch verträglichen Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität von Verbindungen der allgemeinen Formel I zu humanen CGRP-Rezeptoren und ihrer antagonistischen Eigenschaften wurden die folgenden Versuche durchgeführt:

A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, $NaHCO_3$ 16.2, $MgSO_4$ 0.8, $NaHPO_4$ 1.0, $CaCl_2$ 1.8, D-Glucose 5.5, HEPES 30, pH7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM $MgCl_2$, 1 mM EDTA, pH 7.40), angereichert mit 1% Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM $MgCl_2$, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM $^{125}$I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit. Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die Verbindungen der allgemeinen Formel I zeigen in dem beschriebenen Test $IC_{50}$-Werte ≤ 10000 nM.

B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen ($10^{-11}$ bis $10^{-6}$ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die $pA_2$-Werte antagonistisch wirkender Substanzen graphisch ermittelt.

Die Verbindungen der allgemeinen Formel I zeigen in dem beschriebenen in-vitro-Testmodell CGRP-antagonistische Eigenschaften in einem Dosisbereich zwischen $10^{-11}$ bis $10^{-5}$ M.

Auf Grund ihrer pharmakologischen Eigenschaften eignen sich die Verbindungen der allgemeinen Formel I und deren Salze mit physiologisch verträglichen Säuren bzw. Basen somit zur akuten und prophylaktischen Behandlung von Kopfschmerzen, insbesondere Migräne- bzw. Cluster-Kopfschmerz. Weiterhin beeinflussen die Verbindungen der allgemeinen Formel I auch die folgenden Erkrankungen positiv: Nicht-insulinabhängigen Diabetes mellitus

("NIDDM"), cardiovaskuläre Erkrankungen, Erkrankungen der Haut, insbesondere thermische und strahlenbedingte Hautschäden inclusive Sonnenbrand, entzündliche Erkrankungen, z.B. entzündliche Gelenkerkrankungen (Arthritis), entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis, sowie Morphintoleranz. Darüber hinaus zeigen die Verbindungen der allgemeinen Formel I eine lindernde Wirkung auf Schmerzzustände im allgemeinen und sind ferner geeignet zur Bekämpfung menopausaler Hitzewallungen.

Die zur Erzielung einer entsprechenden Wirkung erforderliche Dosierung beträgt bei intravenöser oder subcutaner Gabe zweckmäßig 0,0001 bis 3 mg/kg Körpergewicht, vorzugsweise 0,01 bis 1 mg/kg Körpergewicht, und bei oraler, nasaler oder inhalativer Gabe 0,01 bis 10 mg/kg Körpergewicht, vorzugsweise 0,1 bis 10 mg/kg Körpergewicht, jeweils 1 bis 3 x täglich.

Hierzu lassen sich die erfindungsgemäß hergestellten Verbindungen der allgemeinen Formel I, gegebenenfalls in Kombination mit anderen Wirksubstanzen, wie z.B. Antiemetica, Prokinetica, Neuroleptica, Antidepressiva, Neurokinin-Antagonisten, Anticonvulsiva, Histamin-Hl-Rezeptorantagonisten, Antimuscarinica, β-Blockern, α-Agonisten und α-Antagonisten, Ergotalkaloiden, schwachen Analgetica, nichtsteroidalen Antiphlogistica, Corticosteroiden, Calcium-Antagonisten, 5-$HT_{1D}$-Agonisten, 5-$HT_{1F}$-Agonisten oder anderen Antimigränemitteln, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen einarbeiten.

Für die oben erwähnten Kombinationen kommen somit als weitere Wirksubstanzen beispielsweise Meloxicam, Ergotamin, Dihydroergotamin, Metoclopramid, Domperidon, Diphenhydramin, Cyclizin, Promethazin, Chlorpromazin, Dexamethason, Flunarizin, Dextropropoxyphen, Meperidin, Propranolol, Nadolol, Atenolol, Clonidin, Indoramin, Carbamazepin, Phenytoin, Valproat, Amitryptilin, Lidocain, Diltiazem oder Sumatriptan und andere 5-$HT_{1D}$-Agonisten wie z.B. Naratriptan, Zolmitriptan, Avitriptan, Rizatriptan und Eletriptan in Betracht. Die Dosis für diese Wirksubstanzen beträgt hierbei zweckmäßig 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der Verbindungen der allgemeinen Formel I als wertvolle Hilfsmittel zur Erzeugung und Reinigung (Affinitätschromatographie) von Antikörpern sowie, nach geeigneter radioaktiver Markierung, beispielsweise durch direkte Markierung mit $^{125}I$ oder $^{131}I$ oder durch Tritiierung geeigneter Vorstufen, beispielsweise durch Ersatz von Halogenatomen durch Tritium, in RIAund ELISA-Assays und als diagnostische bzw. analytische Hilfsmittel in der Neurotransmitter-Forschung.

Die nachfolgenden Beispiele sollen die Erfindung näher erläutern:

<u>Vorbemerkungen :</u>

Für alle Verbindungen liegen befriedigende Elementaranalysen, IR-, UV-, $^1H$-NMR und in der Regel auch Massenspekten vor. Wenn nicht anders angegeben, wurden $R_f$-Werte unter Verwendung von DC-Fertigplatten Kieselgel 60 $F_{254}$ (E. Merck, Darmstadt, Artikel-Nr. 5729) ohne Kammersättigung bestimmt. Falls nähere Angaben zur Konfiguration fehlen, bleibt offen, ob es sich um reine Enantiomere handelt oder ob partielle oder gar völlige Racemisierung eingetreten ist. Zur Chromatographie wurden die folgenden Fließmittel bzw. Fließmittelgemische verwendet:

FM1 = Dichlormethan/Cyclohexan/Methanol/Ammoniak 7/1.5/1.5/0.2 (v/v/v/v)
FM2 = Dichlormethan/Methanol/Ammoniak 7.5/2.5/0.5 (v/v/v)
FM3 = Dichlormethan/Methanol 8/2 (v/v)
FM4 = Dichlormethan/Essigester/Methanol/Cyclohexan/konz. wässeriges Ammoniak = 59/25/7,5/7,5/1 (v/v/v/v/v)
FM5 = Essigester/Dichlormethan = 7/3 (v/v)
FM6 = Essigester/Petrolether = 1/1 (v/v)
FM7 = Dichlormethan/Methanol/konz. wässeriges Ammoniak = 80/20/1 (v/v/v)

In der Versuchsbeschreibung werden die folgenden Abkürzungen verwendet:

| | |
|---|---|
| Fp.: | Schmelzpunkt |
| (Z): | (Zersetzung) |
| DIEA: | N,N-Diisopropyl-N-ethylamin |
| Boc: | (1,1-Dimethylethoxy)carbonyl |
| TBTU: | 2-(1H-Benzotriazol-1-yl)-1,1,3, 3-tetramethyluroniumtetrafluoroborat |

HOBt: 1-Hydroxybenzotriazol-hydrat
CDT: 1,1'-Carbonyldi-(1,2,4-triazol)
THF: Tetrahydrofuran
DMF: Dimethylformamid
Fmoc: (9-Fluorenylmethoxy)carbonyl
EE: Essigsäureethylester
PE: Petrolether
LM: Lösemittel

Beispiel 1

1-[4-Amino-3,5-dibrom-N-[[4-(3,4-dihydro-2(1H)-oxochinazolin-3-yl)-1-piperidinyl]methylsulfonyliminomethyl]-D-phenylalanyl]-4-(1-piperidinyl)-piperidin

a) 1-[4-Amino-3,5-dibrom-N-[(phenoxy)methylsulfonyliminomethyl]-D-phenylalanyl]-4-(1-piperidinyl)-piperidin

Das Gemisch aus 0.5g (1.716 mMol) N-Methansulfonylimino-diphenylcarbonat, 0.72 g (1.005 mMol) 1-(4-Amino-3,5-dibrom-D-phenylalanyl)-4-(1-piperidinyl)piperidin-bis-(trifluoracetat), 0.5 ml (3.0 mMol) DIEA und 50 ml Dichlormethan wurde 1 Stunde bei Zimmertemperatur gerührt, dann im Vakuum eingeengt, erneut in 50 ml Dichlormethan aufgenommen, nacheinander mit je 20 ml 0.5 N Natronlauge und Wasser gewaschen, über Magnesiumsulfat getrocknet und im Vakuum eingedampft. Das in einer Ausbeute von 0.67 g (97 % der Theorie) erhaltene Rohprodukt wurde ohne weitere Reinigung in der folgenden Stufe verwendet.

b) 1-[4-Amino-3,5-dibrom-N-[[4-(3,4-dihydro-2(1H)-oxochinazolin-3-yl)-1-piperidinyl]methylsulfonyliminomethyl]-D-phenylalanyl]-4-(1-piperidinyl)-piperidin

Das Gemisch aus 0.4 g (0.584 mMol) 1-[4-Amino-3,5-dibrom-N-[(phenoxy)methylsulfonyliminomethyl]-D-phenylalanyl]-4-(1-piperidinyl)-piperidin, 0.46 g (1.989 mMol) 3,4-Dihydro-3-(4-piperidinyl)-2(1H)-chinazolinon und 10 ml 2-Pentanol wurde 14 Stunden lang unter Rückfluß gekocht. Das Gemisch wurde im Vakuum eingeengt, der Rückstand an Kieselgel (MN-Kieselgel 60, Macherey-Nagel, 30-60 µm) unter Verwendung von anfangs Dichlormethan, dann Methanol/konz. Ammoniak (9/1 v/v) zum Eluieren säulenchromatographisch gereinigt. Nach üblicher Aufarbeitung erhielt man 130 mg (27 % der Theorie) eines amorphen, farblosen Festproduktes.

IR (KBr) : 1664 $cm^{-1}$ (C=O)
$R_f$: 0.53 (FM1)
ESI-MS: $(M+H)^+$ = 821/823/825 ($Br_2$)

Beispiel 2

1-[4-Amino-3,5-dibrom-N-[[4-(3,4-dihydro-2(1H)-oxochinazolin-3-yl)-1-piperidinyl]cyaniminomethyl]-D-phenylalanyl]-4-(1-piperidinyl)-piperidin

a) 1-[4-Amino-3,5-dibrom-N-[(phenoxy)cyaniminomethyl]-D-phenylalanyl]-4-(1-piperidinyl)-piperidin

Hergestellt analog Beispiel 1a) aus N-Cyan-iminodiphenylcarbonat und 1-(4-Amino-3,5-dibrom-D-phenylalanyl)-4-(1-piperidinyl)piperidin-bis-(trifluoracetat) in quantitativer Ausbeute. Das erhaltene Rohprodukt wurde ohne weitere Reinigung in der folgenden Stufe verwendet.

b) 1-[4-Amino-3,5-dibrom-N-[[4-(3,4-dihydro-2(1H)-oxochinazolin-3-yl)-1-piperidinyl]cyaniminomethyl]-D-phenylalanyl]-4-(1-piperidinyl)-piperidin

Hergestellt analog Beispiel 1b) aus 3,4-Dihydro-3-(4-piperidinyl)-2(1H)-chinazolinon und 1-[4-Amino-3,5-dibrom-N-[(phenoxy)cyaniminomethyl]-D-phenylalanyl]-4-(1-piperidinyl)-piperidin in einer Ausbeute von 43 % der Theorie. Farblose, amorphe Substanz.

IR (KBr) : 1664 (C=O), 2173 (CN) $cm^{-1}$
$R_f$: 0.48 (FM1)
ESI-MS: $(M+H)^+$ = 768/770/772 ($Br_2$)

Beispiel 3

1-[4-Amino-3,5-dibrom-N-[[4-(3,4-dihydro-2(1H)-oxochinazolin-3-yl)-1-piperidinyl]phenylsulfonyliminomethyl]-D-phenylalanyl]-4-(1-piperidinyl)-piperidin

a) 1-[4-Amino-3,5-dibrom-N-[(phenoxy)phenylsulfonyliminomethyl]-D-phenylalanyl]-4-(1-piperidinyl)-piperidin

Hergestellt analog Beispiel 1a) aus N-Benzensulfonyl-iminodiphenylcarbonat und 1-(4-Amino-3,5-dibrom-D-phenylalanyl)-4-(1-piperidinyl)piperidin-bis-(trifluoracetat) in einer Ausbeute von 60 % der Theorie. Farblose, amorphe Substanz vom $R_f$ 0.41 (Fließmittel: Dichlormethan/Methanol/konz. Ammoniak 9/1/0.1).

b) 1-[4-Amino-3,5-dibrom-N-[[4-(3,4-dihydro-2(1H)-oxochinazolin-3-yl)-1-piperidinyl]phenylsulfonyliminomethyl]-D-phenylalanyl]-4-(1-piperidinyl)-piperidin

Hergestellt analog Beispiel 1b) aus 3,4-Dihydro-3-(4-piperidinyl)-2(1H)-chinazolinon und 1-[4-Amino-3,5-dibrom-N-[(phenoxy)phenylsulfonyliminomethyl]-D-phenylalanyl]-4-(1-piperidinyl)-piperidin in einer Ausbeute von 33 % der Theorie. Farblose, amorphe Substanz.

| | |
|---|---|
| IR (KBr): | 1664 $cm^{-1}$ (C=O) |
| $R_f$: | 0.50 (FM1) |
| ESI-MS: | $(M+H)^+$ = 883/885/887($Br_2$) |

Beispiel 4

1-[3,5-Dibrom-N-[[4-(3,4-dihydro-2(1H)-oxochinazolin-3-yl)-1-piperidinyl]cyaniminomethyl]-D-tyrosyl]-4-(1-piperidinyl)-piperidin

a) 1-[3,5-Dibrom-N-[(phenoxy)cyaniminomethyl]-D-tyrosyl]-4-(1-piperidinyl)-piperidin

Hergestellt analog Beispiel 1a) aus N-Cyanimino-diphenylcarbonat und 1-(3,5-Dibrom-D-tyrosyl)-4-(1-piperidinyl)piperidin-bis-(trifluoracetat) in einer Ausbeute von 23 % der Theorie. Nach dem Verreiben mit t-Butylmethylether/Isopropanol (1/1 v/v): Farblose, kristalline Substanz.

b) 1-[3,5-Dibrom-N-[[4-(3,4-dihydro-2(1H)-oxochinazolin-3-yl)-1-piperidinyl]cyaniminomethyl]-D-tyrosyl]-4-(1-piperidinyl)-piperidin

Hergestellt analog Beispiel 1b) aus 3,4-Dihydro-3-(4-piperidinyl)-2(1H)-chinazolinon und 1-[3,5-Dibrom-N-[(phenoxy)cyaniminomethyl]-D-tyrosyl]-4-(1-piperidinyl)-piperidin in einer Ausbeute von 20 % der Theorie. Farblose, amorphe Substanz.

| | |
|---|---|
| IR (KBr): | 1658 (C=O), 2173 (CN) $cm^{-1}$ |
| $R_f$: | 0.28 (FM1) |
| ESI-MS: | $(M+H)^+$ = 769/771/773($Br_2$) |

Beispiel 5

1-[$N^2$-[3,5-Dibrom-N-[[4-(3,4-dihydro-2(1H)-oxochinazolin-3-yl)-1-piperidinyl]methylsulfonyliminomethyl]-D-tyrosyl]-L-lysyl]-4-(4-pyridinyl)-piperazin

a) 1-[$N^2$-[3,5-Dibrom-N-[[4-(3,4-dihydro-2(1H)-oxochinazolin-3-yl)-1-piperidinyl]methylsulfonyliminomethyl]-D-tyrosyl] -$N^6$-(1,1-dimethylethoxycarbonyl)-L-lysyl]-4-(4-pyridinyl)-piperazin

Zu der Lösung von 1.0 g (1.402 mMol) 1-[$N^2$-[3,5-Dibrom-D-tyrosyl] -$N^6$-(1,1-dimethylethoxycarbonyl)-L-lysyl]-4-(4-pyridinyl)-piperazin in 50 ml Dioxan gab man 0.4 g (1.373 mMol) N-(Methansulfonyl)-iminodiphenylcarbonat und rührte 2 Stunden bei Zimmertemperatur. Nach vollständiger Umsetzung (DC) setzte man 0.33 g (1.427 mMol) 3,4-Dihydro-3-(4-piperidinyl)-2(1H)-chinazolinon zu und kochte 6 Stunden unter Rückfluß. Das Reaktionsgemisch wurde im Vakuum eingedampft, der verbleibende Rückstand an Kieselgel (MN-Kieselgel 60, Macherey-Nagel, 30-60 µm) unter Verwendung von anfangs Dichlormethan, dann Methanol/konz. Ammoniak (9/1 v/v) zum Eluieren säulenchromatographisch gereinigt. Nach üblicher Aufarbeitung erhielt man 590 mg (41 % der Theorie) eines amorphen, farblosen Festproduktes.

IR (KBr): 1655 $cm^{-1}$ (C=O)

ESI-MS: $(M+H)^+$ = 1045/1047/1049($Br_2$)

b) 1-[$N^2$-[3,5-Dibrom-N-[[4-(3,4-dihydro-2(1H)-oxochinazolin-3-yl)-1-piperidinyl]methylsulfonyliminomethyl]-D-tyrosyl]-L-lysyl]-4-(4-pyridinyl)-piperazin

Zu einer Mischung aus 0.58 g (0.554 mmol) 1-[$N^2$-[3,5-Dibrom-N-[[4-(3,4-dihydro-2(1H)-oxochinazolin-3-yl)-1-piperidinyl]methylsulfonyliminomethyl]-D-tyrosyl]-$N^6$-(1,1-dimethylethoxycarbonyl)-L-lysyl]-4-(4-pyridinyl)-piperazin in 20 ml Methylenchlorid wurden 10 ml Trifluoressigsäure zugegeben. Das Reaktionsgemisch wurde 3 Stunden bei Raumtemperatur gerührt und anschließend im Vakuum eingeengt. Der verbleibende Rückstand wurde in 50 ml Wasser aufgenommen, mit festem Natriumhydrogencarbonat vorsichtig alkalisch gestellt. Der ausgefallene Niederschlag wurde abgenutscht, mit Wasser und dann mit t-Butylmethylether gut gewaschen, schließlich an der Luft getrocknet. Man erhielt 0.36 g (69 % der Theorie)eines farblosen, amorphen Feststoffs. .

IR (KBr) : 1649 .$cm^{-1}$ (C=O)
$R_f$: 0.07 (FM1)
ESI-MS: $(M+H)^+$ = 945/947/949 ($Br_2$)

Beispiel 6

1-[$N^2$-[3,5-Dibrom-N-[[4-(3,4-dihydro-2(1H)-oxochinazolin-3-yl)-1-piperidinyl]phenylsulfonyliminomethyl]-D-tyrosyl]-L-lysyl]-4-(4-pyridinyl)-piperazin

a) 1-[$N^2$-[3,5-Dibrom-N-[[4-(3,4-dihydro-2(1H)-oxochinazolin-3-yl)-1-piperidinyl]phenylsulfonyliminomethyl]-D-tyrosyl]-$N^6$-(1,1-dimethylethoxycarbonyl)-L-lysyl] -4-(4-pyridinyl)-piperazin

Hergestellt analog Beispiel 5a) aus N-(Benzensulfonyl)-iminodiphenylcarbonat, 3,4-Dihydro-3-(4-piperidinyl)-2(1H)-chinazolinon und 1-[$N^2$-(3,5-Dibrom-D-tyrosyl)-$N^6$- (1,1-dimethylethoxycarbonyl)-L-lysyl]-4-(4-pyridinyl)-piperazin in einer Ausbeute von 43 % der Theorie. Farblose, amorphe Substanz.

IR (KBr): 1657 .$cm^{-1}$ (C=O)
ESI-MS: $(M+H)^+$ = 1107/1109/1111 ($Br_2$)
$(M+H+Na)^{++}$ = 565/566/567 ($Br_2$)

b) 1-[$N^2$-[3,5-Dibrom-N-[[4-(3,4-dihydro-2(1H)-oxochinazolin-3-yl)-1-piperidinyl]phenylsulfonyliminomethyl]-D-tyrosyl]-L-lysyl]-4-(4-pyridinyl)-piperazin

Hergestellt analog Beispiel 5b) aus 1-[$N^2$-[3,5-Dibrom-N-[[4-(3,4-dihydro-2(1H)-oxochinazolin-3-yl)-1-piperidinyl]-phenylsulfonyliminomethyl]-D-tyrosyl]-$N^6$-(1,1-dimethylethoxycarbonyl)-L-lysyl]-4-(4-pyridinyl)-piperazin und Trifluoressigsäure in einer Ausbeute von 91 % der Theorie. Farblose, amorphe Substanz.

IR (KBr): 1649 (C=O) $cm^{-1}$
$R_f$: 0.13 (FM1)
ESI-MS: $(M+H)^+$ = 1007/1009/1111($Br_2$)

Beispiel 7

1-[4-Amino-3,5-dibrom-N-[[4-(3,4-dihydro-2(1H)-oxochinazolin-3-yl)-1-piperidinyl]cyaniminomethyl]-D-phenylalanyl]-4-(1-methyl-4-piperidinyl)-piperidin

Die Mischung aus 0.35 g (1.469 mMol) N-Cyan-iminodiphenylcarbonat, 0.75 g (1.493 mMol) 1- [4-Amino-3,5-dibrom-D-phenylalanyl]-4-(1-methyl-4-piperidinyl)-piperidin und 30 ml wasserfreiem Dichlormethan wurde 14 Stunden bei Zimmertemperatur gerührt. Das Reaktionsgemisch wurde, zuletzt im Vakuum, vom Lösemittel befreit, der Rückstand mit 0.35 g (1.513 mMol) 3,4-Dihydro-3-(4-piperidinyl)-2(1H)-chinazolinon und 20 ml 2-Pentanol versetzt

und 24 Stunden unter Rückfluß gekocht. Das Gemisch wurde im Vakuum eingeengt, der Rückstand an Kieselgel (MN-Kieselgel 60, Macherey-Nagel, 30-60 µm) unter Verwendung von anfangs Dichlormethan, dann Methanol/konz. Ammoniak (9/1 v/v) zum Eluieren säulenchromatographisch gereinigt. Nach üblicher Aufarbeitung erhielt man 700 mg (61 % der Theorie) eines amorphen, farblosen Festproduktes.

IR (KBr): 1668 (C=O), 2173 (CN) $cm^{-1}$
$R_f$: 0.87 (Fließmittel: Dichlormethan/Methanol/konz. Ammoniak 80/20/2 v/v/v)
ESI-MS: $(M+H)^+$ = 782/784/786 ($Br_2$)

Beispiel 8

1-[4-Brom-N-[[4-(3,4-dihydro-2(1H)-oxochinazolin-3-yl)-1-piperidinyl]cyaniminomethyl]-3,5-dimethyl-D, L-phenylalanyl]-4-(1-piperidinyl)-piperidin

a) 1-[4-Brom-N-[(phenoxy)cyaniminomethyl]-3,5-dimethyl-D, L-phenylalanyl]-4-(-1-piperidinyl)-piperidin

Hergestellt analog Beispiel 1a), jedoch unter Verwendung von Dioxan als Lösemittel, aus N-Cyan-iminodiphenylcarbonat und 1- (4-Brom-3,5-dimethyl-D,L-phenylalanyl)-4-(1-piperidinyl)-piperidin-bis-(trifluoracetat) in einer Ausbeute von 51 % der Theorie. Farblose, amorphe Substanz.

b) 1-[4-Brom-N-[[4-(3,4-dihydro-2(1H)-oxochinazolin-3-yl)-1-piperidinyl]cyaniminomethyl]-3,5-dimethyl-D,L-phenylalanyl]-4-(1-piperidinyl)-piperidin

Hergestellt analog Beispiel 1b) aus 3,4-Dihydro-3-(4-piperidinyl)-2(1H)-chinazolinon und 1-[4-Brom-N-[(phenoxy)cyaniminomethyl]-3, 5-dimethyl-D,L-phenylalanyl]-4-(1-piperidinyl)-piperidin in einer Ausbeute von 45 % der Theorie. Farblose, amorphe Substanz.

IR (KBr): 1664 (C=O) , 2173 (CN) $cm^{-1}$
$R_f$: 0.37 (Fließmittel: Dichlormethan/Ethylacetat/Cyclohexan/Methanol/konz. Ammoniak 60/16/5/5/0.6 v/v/v/v/v)
MS: $M^+$ = 702/704 ($Br_2$)

Beispiel 9

1-[3,5-Dibrom-N-[[4-(3,4-dihydro-2(1H)-oxochinazolin-3-yl)-1-piperidinyl]cyaniminomethyl]-D-tyrosyl]-4-(4-pyridinyl)-piperazin

Hergestellt analog Beispiel 1b) aus 3,4-Dihydro-3-(4-piperidinyl)-2(1H)-chinazolinon und 1-[3,5-Dibrom-N-[(phenoxy)cyaniminomethyl]-D-tyrosyl]-4-(4-pyridinyl)-piperazin in einer Ausbeute von 10 % der Theorie. Farblose, amorphe Substanz.

IR (KBr): 1657 (C=O), 2171 (CN) $cm^{-1}$

$R_f$: 0.68 (Fließmittel: Methanol)

ESI-MS: $(M+H)^+$ = 764/766/768 ($Br_2$)

Beispiel 10

1-[4-Amino-3,5-dibrom-N-[[4-(3,4-dihydro-2(1H)-oxochinazolin-3-yl)-1-piperidinyl]cyaniminomethyl]-D-phenylalanyl]-4-(4-pyridinyl)-piperazin

Hergestellt analog Beispiel 7 aus N-Cyano-iminodiphenylcarbonat, 3,4-Dihydro-3-(4-piperidinyl)-2(1H)-chinazolinon und 1- [4-Amino-3,5-dibrom-D-phenylalanyl]-4-(4-pyridinyl)-piperazin in einer Ausbeute von 43 % der Theorie. Farblose, amorphe Substanz.

IR (KBr) : 1660 (C=O), 2171 (CN) $cm^{-1}$
$R_f$: 0.27 (Fließmittel: Dichlormethan/Methanol/konz. Ammoniak 9/1/0.1 v/v/v)

ESI-MS: $(M+H)^+$ = 763/765/767 ($Br_2$)

Beispiel 11

1-[3,5-Dibrom-N-[[4-(3,4-dihydro-2(1H)-oxochinazolin-3-yl)-1-piperidinyl]cyaniminomethyl]-D-tyrosyl]-4-(1-methyl-4-piperidinyl)-piperidin

Hergestellt analog Beispiel 7 aus N-Cyano-iminodiphenylcarbonat, 3,4-Dihydro-3- (4-piperidinyl)-2(1H)-chinazolinon und 1-[3,5-Dibrom-D-tyrosyl]-4-(4-pyridinyl)-piperazin in einer Ausbeute von 12 % der Theorie. Farblose, amorphe Substanz.

IR (KBr): 2175 (CN) $cm^{-1}$
$R_f$: 0.22 (Fließmittel Dichlormethan/Methanol/konz. Ammoniak 8/2/0.2 v/v/v)
ESI-MS: $(M+H)^+$ = 783/785/787 ($Br_2$)

Beispiel 12

1-[3,5-Dibrom-N-[[4-[3,4-dihydro-2(1H)-oxochinazolin-3-yl]-1-piperidinyl]cyaniminomethyl]-D-tyrosyl]-4-(4-methyl-1-piperazinyl)piperidin

Hergestellt analog Beispiel 7 aus N-Cyano-iminodiphenylcarbonat, 3,4-Dihydro-3-(4-piperidinyl)-2(1H)-chinazolinon und 1-[3,5-Dibrom-D-tyrosyl]-4-(4-methyl-1-piperazinyl)-piperidin in einer Ausbeute von 13 % der Theorie. Farblose, amorphe Substanz.

IR (KBr): 1674 (C=O), 2173 (CN) $cm^{-1}$
$R_f$ : 0.30 (Fließmittel Dichlormethan/Methanol/konz. Ammoniak 8/2/0.2 v/v/v)
ESI-MS: $(M+H)^+$ = 784/786/788 ($Br_2$)

Beispiel 13

1-[4-Brom-N-[[4-(3,4-dihydro-2(1H)-oxochinazolin-3-yl)-1-piperidinyl]cyaniminomethyl]-3 ,5-dimethyl-D, L-phenylalanyl]-4-(1-methyl-4-piperidinyl)-piperidin

Hergestellt analog Beispiel 7 aus N-Cyano-iminodiphenylcarbonat, 3,4-Dihydro-3-(4-piperidinyl)-2(1H)-chinazolinon und 1-[4-Brom-3,5-dimethyl-D-phenylalanyl]-4-(1-methyl-4-piperidinyl)-piperidin in einer Ausbeute von 44 % der Theorie. Farblose, amorphe Substanz.

IR (KBr): 1666 (C=O) , 2173 (CN) $cm^{-1}$
$R_f$: 0.63 (Fließmittel: Dichlormethan/Cyclohexan/Metha nol/konz. Ammoniak 70/15/15/2 v/v/v/v)
MS: $M^+$ = 716/718 ($Br_2$)

Beispiel 14

1-[4-Amino-3,5-dibrom-N-[[4-[1,3-dihydro-4-phenyl-2(2H)-oxoimidazol-1-yl]-1-piperidinyl]cyaniminomethyl]-D-phenylalanyl]-4-(4-methyl-1-piperazinyl)-piperidin

a) 1-[4-Amino-3,5-dibrom-N-[(phenoxy)cyaniminomethyl]-D-phenylalanyl]-4-(4-methyl-1-piperazinyl)-piperidin

Hergestellt analog Beispiel 1a) aus N-Cyan-iminodiphenylcarbonat und 1-(4-Amino-3,5-dibrom-D-phenylalanyl)-4-(4-methyl-1-piperazinyl)piperidin-bis-(trifluoracetat) in einer Ausbeute von 82 % der Theorie. Farblose, kristalline Substanz vom $R_f$ 0.56 (FM1).

IR (KBr) : 1610 (C=O), 2195 (CN) $cm^{-1}$
ESI-MS: $(M+H)^+$ = 646/648/650 ($Br_2$)

b) 1- [4-Amino-3,5-dibrom-N-[[4-[1,3-dihydro-4-phenyl-2(2H)-oxoimidazol-1-yl]-1-piperidinyl]cyaniminomethyl] -D-phenylalanyl]-4-(4-methyl-1-piperazinyl)-piperidin

Hergestellt analog Beispiel 1b) aus 1,3-Dihydro-1-(4-piperidinyl)-4-phenyl-2(2H)-imidazolon und 1-[4-Amino-3, 5-dibrom-N-[(phenoxy)cyaniminomethyl]-D-phenylalanyl]-4-(4-methyl-1-piperazinyl)-piperidin in einer Ausbeute von 19 % der Theorie. Farblose, amorphe Substanz.

IR (KBr): 1699 (C=O), 2173 (CN) $cm^{-1}$
$R_f$: 0.15 (Fließmittel: Dichlormethan/Methanol/konz. Ammoniak 9/1/0.1 v/v/v))
ESI-MS: $(M+H)^+$ = 796/798/800 ($Br_2$)

Beispiel 15

1-[4-Amino-3,5-dibrom-N-[[4-(2,3,4,5-tetrahydro-2(1H)-oxo-1,3-benzodiazepin-3-yl)-1-piperidinyl]cyaniminomethyl] -D-phenylalanyl]-4-(4-methyl-1-piperazinyl)-piperidin

Hergestellt analog Beispiel 1b) aus 3- (1-Piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(4-methyl-1-piperazinyl)-piperidin in einer Ausbeute von 5 % der Theorie. Farblose, amorphe Substanz.

IR (KBr): 1653 (C=O) , 2173 (CN) $cm^{-1}$
$R_f$: 0.27 (Fließmittel: Dichlormethan/Methanol/konz. Ammoniak 9/1/0.1 v/v/v))
ESI-MS: $(M+H)^+$ = 797/799/801 ($Br_2$)

Beispiel 16

1-[4-Amino-3,5-dibrom-N-[[4-(2,4-dihydro-5-phenyl-3(3H)-oxo-1,2,4-triazol-2-yl)-1-piperidinyl]cyaniminomethyl]-D-phenylalanyl]-4-(1-piperidinyl)-piperdin

a) 1- [4-Amino-3,5-dibrom-N-[(phenoxy)cyaniminomethyl]-D-phenylalanyl]-4-(1-piperidinyl)-piperidin

Hergestellt analog Beispiel 1a) aus N-Cyan-iminodiphenylcarbonat und 1-(4-Amino-3,5-dibrom-D-phenylalanyl)-4-(1-piperidinyl)piperidin in einer Ausbeute von 93 % der Theorie. . Farblose, kristalline Substanz vom $R_f$ 0.25 (Fließmittel: Dichlormethan/Methanol 9/1 v/v).

IR (KBr): 1616 (C=O), 2197 (CN) $cm^{-1}$
ESI-MS: $(M+H)^+$ = 631/633/635 ($Br_2$)

b) 1-[4-Amino-3,5-dibrom-N-[[4-(2,4-dihydro-5-phenyl-3(3H)-oxo-1,2,4-triazol-2-yl)-1-piperidinyl]cyaniminomethyl]-D-phenylalanyl]-4-(1-piperidinyl)-piperidin

Hergestellt analog Beispiel 1b) aus 2,4-Dihydro-2-(4-piperidinyl)-5-phenyl-1,2,4-triazol-3(3H)-on und 1-[4-Amino-3,5-dibrom-N-[(phenoxy)cyaniminomethyl]-D-phenylalanyl]-4-(1-piperidinyl)piperidin in einer Ausbeute von 31 % der Theorie. Farblose, amorphe Substanz.

IR (KBr): 1695 (C=O), 2173 (CN) $cm^{-1}$
$R_f$: 0.26 (Fließmittel: Dichlormethan/Methanol/konz. Ammoniak 9/1/0.1 v/v/v)
ESI-MS: $(M+H)^+$ = 781/783/785 ($Br_2$)

Beispiel 17

1-[4-Amino-3,5-dibrom-N-[[4-(2,3,4,5-tetrahydro-2(1H)-oxo-1,3-benzodiazepin-3-yl)-1-piperidinyl]cyaniminomethyl] -D-phenylalanyl]-4-(1-piperidinyl)-piperidin

Hergestellt analog Beispiel 1b) aus 3-(4-Piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(1-piperidinyl)piperidin in einer Ausbeute von 22 % der Theorie. Farblose, kristalline Substanz.

IR (KBr): 1658 (C=O), 2171 (CN) $cm^{-1}$
$R_f$: 0.33 (Fließmittel: Dichlormethan/Methanol/konz. Ammoniak 9/1/0.1 v/v/v)
ESI-MS: $(M+H)^+$ = 782/784/786 ($Br_2$)

Beispiel 18

1-[3,5-Dibrom-N-[[4-(2,4-dihydro-5-phenyl-3(3H)-oxo-1,2,4-triazol-2-yl)-1-piperidinyl]cyaniminomethyl]-D-tyrosyl]-4-(1-piperidinyl)-piperidin

a) 1- [3,5-Dibrom-N-[(phenoxy)cyaniminomethyl]-D-tyrosyl]-4-(1-piperidinyl)-piperidin

Hergestellt analog Beispiel 1a) aus N-Cyan-iminodiphenylcarbonat und 1-(3,5-Dibrom-D-tyrosyl)-4-(1-piperidinyl)piperidin in einer Ausbeute von 51 % der Theorie. Farblose, kristalline Substanz vom $R_f$ 0.86 (Fließmittel: Dichlormethan/Methanol/konz. Ammoniak 75/25/5 v/v/v).

ESI-MS: $(M+H)^+$ = 632/634/636 ($Br_2$)
$(M-H)^-$ = 630/632/634 ($Br_2$)

b) 1-[3,5-Dibrom-N-[[4-(2,4-dihydro-5-phenyl-3(3H)-oxo-1,2,4-triazol-2-yl)-1-piperidinyl]cyaniminomethyl]-D-tyrosyl]-4-(1-piperidinyl)-piperidin

Hergestellt analog Beispiel 1b) aus 2,4-Dihydro-2-(4-piperidinyl)-5-phenyl-1,2,4-triazol-3(3H)-on und 1-[3,5-Dibrom-N-[(phenoxy)cyaniminomethyl]-D-tyrosyl]-4-(1-piperidinyl)piperidin in einer Ausbeute von 32 % der Theorie. Farblose, amorphe Substanz.

IR (KBr): 1695 (C=O), 2173 (CN) $cm^{-1}$
$R_f$: 0.33 (FM1)
ESI-MS: $(M+H)^+$ = 782/784/786 ($Br_2$)

Beispiel 19

1-[3,5-Dibrom-N-[[4-(2,3,4,5-tetrahydro-2(1H)-oxo-1,3-benzodiazepin-3-yl)-1-piperidinyl]cyaniminomethyl]-D-tyrosyl]-4-(1-piperidinyl)-piperidin

Hergestellt analog Beispiel 1b) aus 3-(4-Piperidinyl)-2,3,4,5-tetrahydro-1,3-benzodiazepin-2(1H)-on und 1-[4-3,5-Dibrom-N-[(phenoxy)cyaniminomethyl]-D-tyrosyl]-4-(1-piperidinyl)piperidin in einer Ausbeute von 40 % der Theorie. Farblose, amorphe Substanz.

IR (KBr): 1653 (C= O), 2171 (CN) $cm^{-1}$
$R_f$: 0.44 (FM1)
ESI-MS: $(M+H)^+$ = 783/785/787 ($Br_2$)

Beispiel 20

1- [3, 5-Dibrom-N-[[4-(2,4-dihydro-5-phenyl-3(3H)-oxo-1,2,4-triazol-2-yl)-1-piperidinyl]cyaniminomethyl]-D-tyrosyl]-4-(1-methyl-4-piperidinyl)-piperazin

a) 1- [3,5-Dibrom-N- [(phenoxy)cyaniminomethyl]-D-tyrosyl]-4-(1-methyl-4-piperidinyl)-piperazin

Hergestellt analog Beispiel 1a) aus N-Cyan-iminodiphenylcarbonat und 1-(3,5-Dibrom-D-tyrosyl)-4-(1-methyl-4-piperidinyl)piperazin in einer Ausbeute von 44 % der Theorie. Farblose, kristalline Substanz vom $R_f$ 0.50 (Fließmittel: Dichlormethan/Methanol/konz. Ammoniak 75/25/5 v/v/v).

IR (KBr): 1622 (C=O) $cm^{-1}$
ESI-MS: $(M+H)^+$ = 647/649/651 ($Br_2$)
$(M-H)^-$ = 645/647/649 ($Br_2$)

b) 1-[3,5-Dibrom-N-[[4-(2,4-dihydro-5-phenyl-3(3H)-oxo-1,2,4-triazol-2-yl)-1-piperidinyl)cyaniminomethyl]-D-tyrosyl]-4-(1-methyl-4-piperidinyl)-piperazin

Hergestellt analog Beispiel 1b) aus ,2,4-Dihydro-2-(4-piperidinyl)-5-phenyl-1,2,4-triazol-3(3H)-on und 1-[3,5-Dibrom-N-[(phenoxy)cyaniminomethyl]-D-tyrosyl]-4-(1-methyl-4-piperidinyl)piperazin in einer Ausbeute von 20 % der Theorie. Farblose, amorphe Substanz.

IR (KBr): 1701 (C=O), 2173 (CN) $cm^{-1}$
$R_f$: 0.18 (Fließmittel Dichlormethan/Methanol/konz. Ammoniak 8/2/0.2 v/v/v)
ESI-MS: $(M+H)^+$ = 797/799/801 ($Br_2$)

Beispiel 21

1-[3,5-Dibrom-N-[[4-(2,3,4,5-tetrahydro-2(1H)-oxo-1,3-benzodiazepin-3-yl)-1-piperidinyl]cyaniminomethyl]-D-tyrosyl]-4-(1-methyl-4-piperidinyl)-piperazin

Hergestellt analog Beispiel 1b) aus 3-(4-Piperidinyl)-2,3,4,5-tetrahydro-1,3-benzodiazepin-2(1H)-on und 1-[3,5-Dibrom-N-[(phenoxy)cyaniminomethyl]-D-tyrosyl]-4-(1-methyl-4-piperidinyl)piperazin in einer Ausbeute von 32 % der Theorie. Farblose, amorphe Substanz.

IR (KBr): 1653 (C=O), 2171 (CN) $cm^{-1}$
$R_f$: 0.19 (Fließmittel Dichlormethan/Methanol/konz. Ammoniak 8/2/0.2 v/v/v)
ESI-MS: $(M+H)^+$ = 798/800/802 ($Br_2$)
$(M-H)^-$ = 796/798/800 ($Br_2$)

Beispiel 22

1- [4-Amino-3,5-dibrom-N-[[4-(2,4-dihydro-5-phenyl-3(3H)-oxo-1,2,4-triazol-2-yl)-1-piperidinyl]cyaniminomethyl]-D-phenylalanyl]-4-(1-methyl-4-piperidinyl)-piperazin

a) 1-[4-Amino-3,5-dibrom-N- [(phenoxy)cyaniminomethyl] -D-phenylalanyl]-4-(1-methyl-4-piperidinyl)-piperazin

Hergestellt analog Beispiel 1a) aus N-Cyan-iminodiphenylcarbonat und 1-(4-Amino-3,5-dibrom-D-phenylalanyl)-4-(1-methyl-4-piperidinyl)piperazin in einer Ausbeute von 38 % der Theorie. Farblose, kristalline Substanz vom $R_f$ 0.57 (FM1).

IR (KBr): 1689 (C=O) $cm^{-1}$
ESI-MS: $(M+H)^+$ = 646/648/650 ($Br_2$)

b) 1- [4-Amino-3,5-dibrom-N-[[4-(2,4-dihydro-5-phenyl-3(3H)-oxo-1,2,4-triazol-2-yl)-1-piperidinyl]cyaniminomethyl]-D-phenylanyl]-4-(1-methyl-4-piperdinyl)-piperazin

Hergestellt analog Beispiel 1b) aus 2,4-Dihydro-2-(4-piperidinyl)-5-phenyl-1,2,4-triazol-3(3H)-on und 1-[4-Amino-3,5-dibrom-N-[(phenoxy)cyaniminomethyl]-D-phenylalanyl]-4-(1-methyl-4-piperidinyl)piperazin in einer Ausbeute von 9 % der Theorie. Farblose, amorphe Substanz.

IR (KBr): 1701 (C=O) , 2171 (CN) $cm^{-1}$
$R_f$: 0.33 (FM1)
ESI-MS: $(M+H)^+$ = 796/798/800 ($Br_2$)

Beispiel 23

1-[4-Amino-3,5-dibrom-N-[(4-(2,3,4,5-tetrahydro-2(1H)-oxo-1,3-benzodiazepin-3-yl)-1-piperidinyl]cyaniminomethyl] -D-phenylalanyl]-4-(1-methyl-4-piperidinyl)piperazin

Hergestellt analog Beispiel 1b) aus 3-(4-Piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(1-methyl-4-piperidinyl)piperazin in einer Ausbeute von 15 % der Theorie. Farblose, amorphe Substanz.

IR (KBr): 2173 (CN) $cm^{-1}$
$R_f$: 0.19 (FM1)
ESI-MS: $(M+H)^+$ = 797/799/801 ($Br_2$)

Beispiel 24

1-[3,5-Dibrom-N-[[4-(3,4-dihydro-2(1H)-oxochinazolin-3-yl)-1-piperidinyl]cyaniminomethyl]-4-methyl-D,L-phenylalanyl]-4-(1-methyl-4-piperidinyl)-piperidin

Hergestellt analog Beispiel 7 aus N-Cyano-iminodiphenylcarbonat, 3,4-Dihydro-3-(4-piperidinyl)-2(1H)-chinazolinon und 1-[3,5-Dibrom-4-methyl-D,L-phenylalanyl] -4-(1-methyl-4-piperidinyl)-piperidin in einer Ausbeute von 49 % der Theorie. Farblose, kristalline Substanz.

IR (KBr): 1668 (C=O), 2175 (CN) $cm^{-1}$
$R_f$: 0.5 (FM1)
ESI-MS: $(M+H)^+$ = 781/783/785 ($Br_2$)
$(M-H)^-$ = 779/781/783 ($Br_2$)

Beispiel 25

1-[3,5-Dibrom-N-[[4-(3,4-dihydro-2(1H)-oxochinazolin-3-yl)-1-piperidinyl]cyaniminomethyl]-4-methyl-D,L-phenylalanyl]-4-(1-piperidinyl)-piperidin

Hergestellt analog Beispiel 7 aus N-Cyano-iminodiphenylcarbonat, 3,4-Dihydro-3-(4-piperidinyl)-2(1H)-chinazolinon und 1-[3,5-Dibrom-4-methyl-D,L-phenylalanyl]-4-(1-piperidinyl)-piperidin in einer Ausbeute von 34 % der Theorie. Farblose, kristalline Substanz.

IR (KBr): 1664 (C=O), 2175 (CN) $cm^{-1}$
$R_f$: 0.55 (FM1)
ESI-MS: $(M+H)^+$ = 767/769/771 ($Br_2$)
$(M-H)^-$ = 765/767/769 ($Br_2$)

Beispiel 26

1- [3,5-Dibrom-N-[[4-(3,4-dihydro-2(1H)-oxochinazolin-3-yl)-1-piperidinyl]cyaniminomethyl]-4-methyl-D,L-phenylalanyl]-4-(4-pyridinyl)-piperazin

Hergestellt analog Beispiel 7 aus N-Cyano-iminodiphenylcarbonat, 3,4-Dihydro-3-(4-piperidinyl)-2(1H)-chinazolinon und 1-[3,5-Dibrom-4-methyl-D,L-phenylalanyl]-4-(4-pyridinyl)-piperazin in einer Ausbeute von 6 % der Theorie. Farblose, kristalline Substanz.

IR (KBr): 2171 (CN) $cm^{-1}$
$R_f$: 0.60 (FM1)
ESI-MS: $(M+H)^+$ = 762/764/766 ($Br_2$)

Beispiel 27

1-[4-Amino-3,5-dibrom-N-[[4-[1,3-dihydro-2(2H)-oxoimidazo-[4,5-c]chinolin-3-yl]-1-piperidinyl]cyaniminomethyl] -Dphenylalanyl]-4-(1-piperidinyl)piperidin

Hergestellt analog Beispiel 1b) aus 1,3-Dihydro-3-(4-piperidinyl)-imidazo[4,5-c]chinolin-2(2H)-on und 1-[4-Amino-3,5-dibrom-N-[(phenoxy)cyaniminomethyl]-D-phenylalanyl]-4-(1-piperidinyl)piperidin in einer Ausbeute von 9 % der Theorie. Farblose, amorphe Substanz.

IR (KBr): 1712 (C=O), 2173 (CN) $cm^{-1}$
$R_f$: 0.45 (FM1)
ESI-MS: $(M+H)^+$ = 805/807/809 ($Br_2$)

Beispiel 28

1- [4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(1-piperidinyl)-piperidin

Hergestellt analog Beispiel 1b) aus 7-Methoxy-3-(4-piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(1-piperidinyl)piperidin in einer Ausbeute von 51 % der Theorie. Farblose Kristalle (aus Aceton).

IR (KBr): 1658 (C=O), 2173 (CN) $cm^{-1}$
$R_f$: 0.65 (FM1)
ESI-MS: $(M+H)^+$ = 812/814/816 ($Br_2$)

Beispiel 29

1- [4-Amino-3,5-dibrom-N-[4-(5,7-dihydro-6-oxodibenzo[d,f]-[1,3]diazepin-5-yl)-1-piperidinyl]cyaniminomethyl]-D-phenylalanyl]-4-(1-piperidinyl)-piperidin

2-Nitro-2'-[[1-(phenylmethyl)-4-piperidinyl]amino]-biphenyl

Zu der Lösung von 30.0 g (0.140 Mol) 2-Amino-2'-nitro-biphenyl und 111.5 g (0.630 Mol)1-(Phenylmethyl)-4-piperidon in 1200 ml Dichlormethan gab man unter Einhaltung einer Reaktionstemperatur von 0°C portionsweise insgesamt 140.5 g (0.630 Mol) Natriumtriacetoxyborhydrid und rührte anschließend 14 Stunden lang bei Zimmertemperatur. Man stellte die Mischung natronalkalisch, trennte die Dichlormethanphase ab, trocknete sie über Natriumsulfat und dampfte sie ein. Der Rückstand wurde mit Methanol digeriert, worauf man filtrierte. Das Filtrat wurde eingedampft, der verbleibende Rückstand an Aluminiumoxid (Aktivitätsstufe 3) unter Verwendung von PE/EE 9/1 (v/v) als Eluens chromatographisch gereinigt. Entsprechende Fraktionen wurden vereinigt, vom Lösemittel befreit und ohne weitere Reinigung in der nächsten Stufe verwendet. Ausbeute: 40.0 g (74 % der Theorie).

2-Amino-2'-[[1-(phenylmethyl)-4-piperidinyl]amino]-biphenyl

Die Lösung von 40.0 g (0.103 Mol) 2-Nitro-2'-[[1-(phenylmethyl)-4-piperidinyl]amino]-biphenyl in 500 ml Methanol wurde in Gegenwart von 5proz. wasserfeuchtem Rhodium auf Kohle 2 Stunden lang hydriert. Der Katalysator wurde abfiltriert, die erhaltene Lösung eingedampft und das so erhaltene Rohprodukt ohne weitere Reinigung in der nächsten Stufe verwendet. Ausbeute: 36.0 g (98 % der Theorie).

c) 5,7-Dihydro-5-[1-(phenylmethyl)-4-piperidinyl]-dibenzo-[d,f] [1,3]diazepin-6-on

Zu der Lösung von 36.0 g (0.101 Mol) 2-Amino-2'-[[1-(phenylmethyl)-4-piperidinyl]amino]-biphenyl in 200 ml Dimethylformamid gab man 40.5 g (0.250 Mol) N,N'-Carbonyl-diimidazol, rührte anschließend das Gemisch 2 Stunden lang bei 100°C, wonach das Lösemittel im Vakuum entfernt wurde. Der Rückstand wurde mit Wasser verrührt, danach mit Dichlormethan erschöpfend extrahiert. Die vereinigten organischen Extrakte wurden über Natriumsulfat getrocknet, mit Aktivkohle geklärt und eingedampft. Der Rückstand (60 g) wurde an Aluminiumoxid der Aktivitätsstufe 3 unter Verwendung von PE/EE 2/1 (v/v) chromatographisch gereinigt. Aus den entsprechenden Fraktionen erhielt man die gesuchte Verbindung in einer Ausbeute von 6.2 g (16 % der Theorie).

IR (KBr): 1676 (C=O) $cm^{-1}$
$R_f$: 0.35 (Fließmittel: Dichlormethan/Methanol = 9.5/0.5 v/v)
MS: $M^+$ = 383

d) 5,7-Dihydro-5-(4-piperidinyl)-dibenzo[d,f] [1,3]diazepin-6-on

Die Lösung von 6.0 g (0.016 Mol) 5,7-Dihydro-5-[1-(phenylmethyl)-4-piperidinyl]-dibenzo(d, f] [1,3] diazepin-6-on in 200 ml Methanol wurde in Gegenwart von 1.5 g 10-proz. Palladiumkohle bis zur Beendigung der Wasserstoffaufnahme bei 50°C hydriert. Nach Entfernung des Katalysators und des Lösemittels erhielt man 3.5 g (76 % der Theorie) der gesuchten Verbindung, die ohne weitere Reinigung in die nächste Stufe eingesetzt wurde.

IR (KBr) : 1678 (C=O) $cm^{-1}$

$R_f$: 0.15 (FM1)
MS: $M^+ = 293$

e) 1-[4-Amino-3,5-dibrom-N-[[4-(5,7-dihydro-6-oxodibenzo-[d,f] [1,3]diazepin-5-yl)-1-piperidinyl]cyaniminomethyl] -Dphenylalanyl]-4-(1-piperidinyl)-piperidin

Hergestellt analog Beispiel 1b) aus 5,7-Dihydro-5-(4-piperidinyl)-dibenzo[d,f] [1,3]diazepin-6-on und 1-[4-Amino-3,5-dibrom-N-[(phenoxy)cyaniminomethyl]-D-phenylalanyl]-4-(1-piperidinyl)piperidin in einer Ausbeute von 25 % der Theorie. Farblose Kristalle.

IR (KBr): 1684 (C=O), 2173 (CN) $cm^{-1}$
$R_f$: 0.65 (FM1)
ESI-MS: $(M+H)^+ = 830/832/834$ ($Br_2$)

Beispiel 30

1-[4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(1-methyl-4-piperidinyl)-piperazin

Hergestellt analog Beispiel 1b) aus 7-Methoxy-3-(4-piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(1-methyl-4-piperidinyl)piperazin in einer Ausbeute von 53 % der Theorie. Farblose Kristalle (Diisopropylether).

IR (KBr) 1647 (C=O) $cm^{-1}$
$R_f$: 0.75 (Fließmittel: Dichlormethan/Methanol/konz. Ammoniak 70/25/5 v/v/v)
ESI-MS: $(M+H)^+ = 827/829/831$ ($Br_2$)
$(M-H)^- = 825/827/829$ ($Br_2$)

Beispiel 31

1-[4-Amino-3,5-dibrom-N-[[4-[1,3-dihydro-2(2H)-oxoimidazo-[4,5-c]chinolin-3-yl]-1-piperidinyl]cyaniminomethyl]-D-phenylalanyl]-4-(1-methyl-4-piperidinyl)piperazin

Hergestellt analog Beispiel 1b) aus 1,3-Dihydro-3-(4-piperidinyl)-imidazo[4,5-c]chinolin-2(2H)-on und 1-[4-Amino-3,5-dibrom-N-[(phenoxy)cyaniminomethyl]-D-phenylalanyl]-4-(1-methyl-4-piperidinyl)piperazin in einer Ausbeute von 13 % der Theorie. Farblose, kristalline Substanz.

IR (KBr) : 1711 (C=O) $cm^{-1}$
$R_f$: 0.70 (Fließmittel: Dichlormethan/Methanol/konz.Ammoniak 70/25/5 v/v/v)
ESI-MS: $(M+H)^+ = 820/822/824$ ($Br_2$)
$(M-H)^- = 818/820/822$ ($Br_2$)

Beispiel 32

1-[4-Amino-3,5-dibrom-N-[[4-(2,3,4,5-tetrahydro-2(1H)-oxo-1,3-benzodiazepin-3-yl)-1-piperidinyl]sulfonyl]-D-phenylalanyl]-4-(1-methyl-4-piperidinyl)-piperazin

a) 1-[4-Amino-3,5-dibrom-N-(2-hydroxyphenoxysulfonyl)-D-phenylalanyl]-4-(1-methyl-4-piperidinyl)-piperazin

Unter äußerer Kühlung mit Eis gab man zu der Lösung von 2.000 g (3.974 mMol) 1-(4-Amino-3,5-dibrom-D-phenylalanyl)-4-(1-methyl-4-piperidinyl)-piperazin und 0.61 ml (0.004 mMol) Triethylamin in 50 ml Dimethylformamid 1.377 g (7.998 mMol) Brenzkatechinsulfat und rührte anschließend 1 Stunde unter weiterer Eiskühlung und 2 Stunden lang bei Zimmertemperatur. Das Reaktionsgemisch wurde bei einer maximalen Badtemperatur von +40°C im Vakuum eingeengt, der Rückstand mit Diethylether verrieben, an der Luft getrocknet und ohne weitere Reinigung in der nächsten Stufe verwendet. Ausbeute: 2.68 g (100 % der Theorie).

$R_f$: 0.30 (FM1)

b)1-[4-Amino-3,5-dibrom-N- [[4-(2,3,4,5-tetrahydro-2(1H)-oxo-1,3-benzodiazepin-3-yl)-1-piperidinyl]sulfonyl]-D-phenylalanyl]-4-(1-methyl-4-piperidinyl)-piperazin

Das Gemisch aus 2.680 g (3.968 mMol) 1-[4-Amino-3,5-dibrom-N-(2-hydroxyphenoxysulfonyl)-D-phenylalanyl]-4-(1-methyl-4-piperidinyl)-piperazin, 1.472 g (6.00 mMol) 3-(4-Piperidinyl)-2,3,4,5-tetrahydro-1,3-benzodiazepin-2(1H)-on und 100 ml Dioxan wurde 1 Stunde unter Rückfluß gekocht, dann im Vakuum eingedampft, der Rückstand in 200 ml 10-proz. wässerige Ammoniaklösung eingerührt. Die so erhaltene Mischung wurde erschöpfend mit Essigsäureethylester extrahiert. Die vereinigten organischen Extrakte wurden über Natriumsulfat getrocknet und vom Lösemittel befreit. Der Rückstand wurde an Kieselgel (MN-Kieselgel 60, Macherey-Nagel, 30-60 μm) unter Verwendung von FM1 als Eluens chromatographisch gereinigt. Die geeigneten Fraktionen wurden vereinigt, eingedampft, der Rückstand mit Diethylether verrieben, abgenutscht und im Vakuum getrocknet. Man erhielt 0.1 g (3.1 % der Theorie) der gesuchten Verbindung in Form farbloser Kristalle vom $R_f$ 0.65 (FM1).

IR (KBr): 1657 (C=O) $cm^{-1}$
ESI-MS: $(M+H)^+$ = 809/811/813 ($Br_2$)

Beispiel 33

1- [3,5-Dibrom-N-[4-(7-methoxy-2,3,4,5-tetrahydro-2(1H)-oxo-1,3-benzodiazepin-3-yl)-1-piperidinyl]cyaniminomethyl] -D-tyrosyl]-4-(1-piperidinyl)-piperidin

Hergestellt analog Beispiel 1b) aus 7-Methoxy-3-(4-piperidinyl)-2,3,4,5-tetrahydro-1,3-benzodiazepin-2(1H)-on und 1-[3,5-Dibrom-N-[(phenoxy)cyaniminomethyl]-D-tyrosyl]-4-(1-piperidinyl)piperidin in einer Ausbeute von 24 % der Theorie. Farblose Kristalle (Diethylether).

IR (KBr): 1653 (C=O) $cm^{-1}$
$R_f$: 0.31 (FM1)
ESI-MS: $(M+H)^+$ = 813/815/817 ($Br_2$)

Beispiel 34

1-[3,5-Dibrom-N-[[4-(7-methoxy-2,3,4,5-tetrahydro-2(1H)-oxo-1,3-benzodiazepin-3-yl)-1-piperidinyl]cyaniminomethyl]-D-tyrosyl]-4-piperidinyl)-piperazin

Hergestellt analog Beispiel 16) aus 7-Methoxy-3-(4-piperidinyl)-2,3,4,5-tetrahydro-1,3-benzodiazepin-2(1H)-on und 1-[3,5-Dibrom-N-[(phenoxy)cyaniminomethyl]-D-tyrosyl]-4-(1-methyl-4-piperidinyl)piperazin in einer Ausbeute von 23 % der Theorie. Farblose Kristalle (Diethylether).

IR (KBr) : 1647 (C=O) $cm^{-1}$
$R_f$: 0.42 (Fließmittel: Dichlormethan/Methanol/konz. Ammoniak 75/25/5 v/v/v)
ESI-MS: $(M-H)^-$ = 826/828/830 ($Br_2$)

Beispiel 35

1-[3, 5-Dibrom-N-[[4-[1,3-dihydro-2(2H)-oxoimidazo[4, 5-c]chinolin-3-yl]-1-piperidinyl]cyaniminomethyl]-D-tyrosyl]-4-(1-piperidinyl)piperidin

Hergestellt analog Beispiel 1b) aus 1,3-Dihydro-3-(4-piperidinyl)-imidazo[4,5-c]chinolin-2(2H)-on und 1-[3,5-Dibrom-N-[(phenoxy)cyaniminomethyl]-D-tyrosyl]-4-(1-piperidinyl)piperidin in einer Ausbeute von 10 % der Theorie. Farblose, kristalline Substanz.

IR (KBr) : 1709 (C=O) $cm^{-1}$
$R_f$: 0.21 (FM1)
ESI-MS: $(M+H)^+$ = 806/808/810 ($Br_2$)
$(M-H)^-$ = 804/806/808 ($Br_2$)

Beispiel 36

1-[3,5-Dibrom-N-[[4-[1,3-dihydro-2(2H)-oxoimidazo[4,5-c]chinolin-3-yl]-1-piperidinyl]cyaniminomethyl]-D-tyrosyl]-4-(1-methyl-4-piperidinyl)piperazin

Hergestellt analog Beispiel 1b) aus 1,3-Dihydro-3-(4-piperidinyl)-imidazo[4,5-c]chinolin-2(2H)-on und 1-[3,5-Dibrom-N-[(phenoxy)cyaniminomethyl]-D-tyrosyl]-4-(1-methyl-4-piperidinyl)piperazin in einer Ausbeute von 31 % der Theorie. Farblose, kristalline Substanz.

IR (KBr) : 1635, 1705 (C=O) $cm^{-1}$
$R_f$: 0.07 (Fließmittel: Dichlormethan/Methanol/konz. Ammoniak 80/20/2 v/ v/v)
ESI-MS: $(M+H)^+$ = 821/823/825 ($Br_2$)
$(M-H)^-$ = 819/821/823 ($Br_2$)

Beispiel 37

1-[4-Amino-3,5-dibrom-N-[[4-(7-methoxy-2,3,4,5-tetrahydro-2(1H)-oxo-1,3-benzodiazepin-3-yl)-1-piperidinyl]cyaniminomethyl]-D-phenylalanyl]-4-(4-methyl-1-piperazinyl)-piperidin

Hergestellt analog Beispiel 1b) aus 7-Methoxy-3-(4-piperidinyl)-2,3,4,5-tetrahydro-1,3-benzodiazepin-2(1H)-on und 1-[4-Amino-3,5-dibrom-N-[(phenoxy)cyaniminomethyl]-D-phenylalanyl]-4-(4-methyl-1-piperazinyl)piperidin in einer Ausbeute von 21 % der Theorie. Farblose Kristalle (Diethylether).

$R_f$: 0.53 (Fließmittel: Dichlormethan/Methanol/konz. Ammoniak 80/20/2 v/v/v)
ESI-MS: $(M+H)^+$ = 827/829/831 ($Br_2$)
$(M-H)^-$ = 825/827/829 ($Br_2$)
$(M+Na)^+$ = 849/851/853 ($Br_2$)

Beispiel 38

1-[4-Amino-3,5-dibrom-N-[4-[1,3-dihydro-2(2H)-oxoimidazo-[4,5-c]chinolin-3-yl]-1-piperidinyl]cyaniminomethyl]-D-phenylalanyl]-4-(4-methyl-1-piperazinyl)piperidin

Hergestellt analog Beispiel 1b) aus 1,3-Dihydro-3-(4-piperidinyl)-imidazo[4,5-c]chinolin-2(2H)-on und 1-[4-Amino-3,5-dibrom-N-[(phenoxy)cyaniminomethyl]-D-phenylalanyl]-4-(4-methyl-1-piperazinyl)piperidin in einer Ausbeute von 8 % der Theorie. Farblose, kristalline Substanz (Diisopropylether).

IR (KBr): 1714 (C=O) $cm^{-1}$
$R_f$ : 0.43 (Fließmittel: Dichlormethan/Methanol/konz. Ammoniak 80/20/2 v/ v/v)
ESI-MS: $(M+H)^+$ = 820/822/824 ($Br_2$)
$(M-H)^-$ = 818/820/822 ($Br_2$)

Die nachfolgenden Beispiele beschreiben die Herstellung pharmazeutischer Anwendungsformen, die als Wirkstoff eine beliebige Verbindung der allgemeinen Formel I enthalten:

Beispiel I

Kapseln zur Pulverinhalation mit 1 mg Wirkstoff

Zusammensetzung:
1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel II

Inhalationslösung für Respimat ® mit 1 mg Wirkstoff

Zusammensetzung:
1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 μl |

Herstellungsverfahren:

Der Wirkstoff und Benzalkoniumchlorid werden in Wasser gelöst und in Respimat®-Kartuschen abgefüllt.

Beispiel III

Inhalationslösung für Vernebler mit 1 mg Wirkstoff

Zusammensetzung:
1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:

Wirkstoff. Natriumchlorid und Benzalkoniumchlorid werden in Wasser gelöst.

Beispiel IV

Treibsgas-Dosieraerosol mit 1 mg Wirkstoff

Zusammensetzung:
1 Hub enthält:

| | |
|---|---|
| Wirkstoff | 1.0 mg |
| Lecithin | 0.1 % |
| Treibgas ad | 50.0 μl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispiel V

Nasalspray mit 1 mg Wirkstoff

Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel VI

Injektionslösung mit 5 mg Wirksubstanz pro 5 ml

Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung :

Glykofurol und Glucose in Wasser für Injektionszwecke auflösen (WfI); Human-Serum-Albumin zugeben; Wirkstoff unter Erwärmen auflösen; mit WfI auf Ansatzvolumen auffüllen; unter Stickstoff-Begasung in Ampullen abfüllen.

Beispiel VII

Injektionslösung mit 100 mg Wirksubstanz pro 20 ml

Zusammensetzung:

| | |
|---|---|
| Wirksubstanz | 100 mg |
| Monokaliumdihydrogenphosphat = $KH_2PO_4$ | 12 mg |
| Dinatriumhydrogenphosphat = $Na_2HPO_4 \cdot 2H_2O$ | 2 mg |
| Natriumchlorid | 180 mg |
| Human-Serum-Albumin | 50 mg |
| Polysorbat 80 | 20 mg |
| Wasser für Injektionszwecke ad | 20 ml |

Herstellung:

Polysorbat 80, Natriumchlorid, Monokaliumdihydrogenphosphat und Dinatriumhydrogenphosphat in Wasser für Injektionszwecke (WfI) auflösen; Human-Serum-Albumin zugeben; Wirkstoff unter Erwärmen auflösen; mit WfI auf Ansatzvolumen auffüllen; in Ampullen abfüllen.

Beispiel VIII

Lyophilisat mit 10 mg Wirksubstanz

Zusammensetzung:

| | |
|---|---|
| Wirksubstanz | 10 mg |
| Mannit | 300 mg |
| Human-Serum-Albumin | 20 mg |

Herstellung :

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (Wfl) auflösen; in Ampullen abfüllen.

Beispiel IX

Tabletten mit 20 mg Wirksubstanz

Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispeil X

Kapseln mit 20 mg Wirksubstanz

Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

Herstellung:

Wirksubstanz, Maisstärke und Kieselsäure homogen mischen; mit Magnesiumstearat mischen; Mischung auf

einer Kapselfüllmaschine in Hartgelatine-Kapseln Größe 3 abfüllen.

Beispiel XI

Zäpfchen mit 50 mg Wirksubstanz

Zusammensetzung:

| | |
|---|---|
| Wirksubstanz | 50 mg |
| Hartfett (Adeps solidus) q.s. ad | 1700 mg |

Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

Beispiel XII

Wäßrige Lösung für die nasale Applikation mit 10 mg Wirksubstanz

Zusammensetzung:

| | |
|---|---|
| Wirksubstanz | 10.0 mg |
| Salzsäure in der zur Bildung eines neutralen Salzes erforderlichen Menge | |
| Parahydroxybenzoesäuremethylester (PHB) | 0.01 mg |
| Parahydroxybenzoesäurepropylester (PHB) | 0.005 mg |
| Wasser gereinigt ad | 1.0 ml |

Herstellung:

Der Wirkstoff wird in gereinigtem Wasser aufgelöst; Salsäure wird zugegeben, bis die Lösung klar wird; PHB-Methyl- und Propylester werden zugegeben; die Lösung wird mit gereinigtem Wasser auf Ansatzvolumen aufgefüllt; die Lösung wird sterilfiltriert und in ein entsprechendes Behältnis abgefüllt.

Beispiel XIII

Wäßrige Lösung für die nasale Applikation mit 5 mg Wirksubstanz

Zusammensetzung:

| | |
|---|---|
| Wirksubstanz | 5 mg |
| 1,2-Propandiol | 300 mg |
| Hydroxyethylcellulose | 5 mg |
| Sorbinsäure | 1 mg |
| Wasser gereinigt ad | 1 ml |

Herstellung:

Der Wirkstoff wird in 1,2-Propandiol gelöst; eine Hydroxyethyl-cellulose-Lösung in gereinigtem Wasser enthaltend Sorbinsäure wird hergestellt und zur Wirkstoff-Lösung gegeben; die Lösung wird sterilfiltriert und in ein entsprechendes Behältnis abgefüllt.

Beispiel XIV

Wäßrige Lösung für die intravenöse Applikation mit 5 mg Wirksubstanz

Zusammensetzung:

| | |
|---|---|
| Wirksubstanz | 5 mg |
| 1,2-Propandiol | 300 mg |
| Mannit | 50 mg |
| Wasser für Injektionszwecke (Wfl) ad | 1 ml |

Herstellung:

Der Wirkstoff wird in 1,2-Propandiol gelöst; die Lösung wird mit Wfl auf annähernd Ansatzvolumen aufgefüllt; das Mannit wird zugegeben und mit Wfl auf Ansatzvolumen aufgefüllt; die Lösung wird sterilfiltriert, in Einzelbehältnisse abgefüllt und autoklaviert.

Beispiel XV

Liposomale Formulierung für die intravenöse Injektion mit 7.5 mg Wirksubstanz

Zusammensetzung:

| | |
|---|---|
| Wirksubstanz | 7.5 mg |
| Ei-lecithin, z.B. Lipoid E 80 | 100.0 mg |
| Cholesterol | 50.0 mg |
| Glycerin | 50.0 mg |
| Wasser für Injektionszwecke ad | 1.0 ml |

Herstellung:

Der Wirkstoff wird in einer Mischung aus Lecithin und Cholesterol gelöst; die Lösung wird zu einer Mischung aus Glycerin und Wfl gegeben und mittels Hochdruck-Homogenisation oder Microfluidizer-Technik homogenisiert; die so erhaltene liposomale Formulierung wird unter aseptischen Bedingungen in ein entsprechendes Behältnis abgefüllt.

Beispiel XVI

Suspension für die nasale Applikation mit 20 mg Wirksubstanz

Zusammensetzung:

| | |
|---|---|
| Wirksubstanz | 20.0 mg |
| Carboxymethylcellulose (CMC) | 20.0 mg |
| Natriummonohydrogenphosphat/Natriumdihydrogenphosphat-Puffer pH 6.8 | q.s. |
| Natriumchlorid | 8.0 mg |
| Parahydroxybenzoesäuremethylester | 0.01 mg |
| Parahydroxybenzoesäurepropylester | 0.003 mg |
| Wasser gereinigt ad | 1.0 ml |

Herstellung:

Der Wirkstoff wird in einer wässrigen CMC-Lösung suspendiert; die anderen Bestandteile werden nacheinander zur Suspension gegeben und die Suspension mit gereinigtem Wasser auf Ansatzvolumen aufgefüllt.

Beispel XVII

Wässrige Lösung für die subcutane Applikation mit 10 mg Wirksubstanz

Zusammensetzung:

| | |
|---|---|
| Wirksubstanz | 10.0 mg |
| Natriummonohydrogenphosphat/Natriumdihydrogenphosphat-Puffer q.s. ad pH | 7.0 |
| Natriumchlorid | 4.0 mg |
| Wasser für Injektionszwecke ad | 0.5 ml |

Herstellung:

Der Wirkstoff wird in der Phosphatpufferlösung gelöst, nach Zugabe des Kochsalz wird mit Wasser auf Ansatzvolumen aufgefüllt. Die Lösung wird sterilfiltriert und nach Abfüllung in ein entsprechendes Behältnis autoklaviert.

Beispiel XVIII

Wässrige Suspension für die subcutane Applikation mit 5 mg Wirksubstanz

Zusammensetzung:

| | |
|---|---|
| Wirksubstanz | 5.0 mg |
| Polysorbat 80 | 0.5 mg |
| Wasser für Injektionszwecke | 0.5 ml |

Herstellung:

Der Wirkstoff wird in der Polysorbat 80-Lösung suspendiert und mittels geeigneter Dispiergiertechnik (z.B. Naßmahlung, Hochdruckhomogenisation, Mikrofluidisierung etc.) auf eine Teilchengröße von ca. 1 μm zerkleinert. Die Suspension wird unter aseptischen Bedingungen in ein entsprechendes Behältnis abgefüllt.

## Patentansprüche

1. Abgewandelte Aminosäureamide der allgemeinen Formel

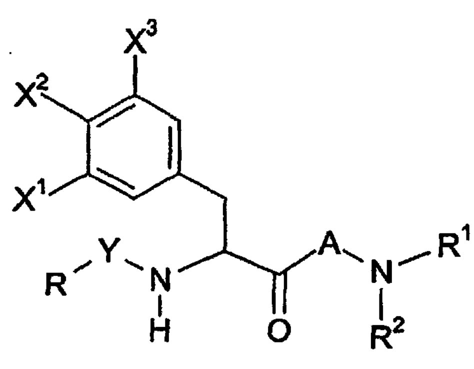

(I),

in der
R die 1-Piperidinylgruppe, die in 4-Stellung durch einen über ein Stickstoffatom gebundenen, einfach oder zweifach ungesättigten 5- bis 7-gliedrigen Aza-, Diaza- oder Triaza-Heterocyclus, der ein oder zwei mit einem Stickstoffatom verknüpfte Carbonylgruppen enthält, substituiert ist,

wobei die vorstehend erwähnten Heterocyclen an einem Kohlenstoffatom durch eine Phenylgruppe substituiert sein können,

eine olefinische Doppelbindung eines der vorstehend erwähnten ungesättigten Heterocyclen mit einem Benzol-, Pyridin- oder Chinolin-Ring kondensiert sein kann oder zwei olefinische Doppelbindungen in einem der vorstehend erwähnten ungesättigten Heterocyclen benzokondensiert sein können,

und wobei die vorstehend erwähnten kondensierten Heterocyclen im Kohlenstoffgerüst und/oder an den in diesen Gruppen enthaltenen Phenylgruppen durch Fluor-, Chlor- oder Bromatome, durch $C_{1-3}$-Alkyl-, Trifluormethyl-, $C_{1-3}$-Alkoxy-, Hydroxy-, Amino-, Nitro-, Phenyl-, Carboxy-, Methoxycarbonyl-, Ethoxycarbonyl-, Aminocarbonyl-, Methylaminocarbonyl-, Hydroxyethylaminocarbonyl-, (4-Morpholinyl)carbonyl-, (1-Piperidinyl)carbonyl- oder (4-Methyl-1-piperazinyl)carbonylgruppen mono-, di- oder trisubstituiert sein können,

wobei die Substituenten gleich oder verschieden sein können und eine Mehrfachsubstitution mit den drei letztgenannten Substituenten ausgeschlossen ist,

Y die zweiwertigen Reste

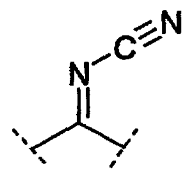 , $-SO_2-$ oder 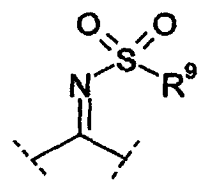

worin $R^9$ einen $C_{1-3}$-Alkylrest oder einen gegebenenfalls durch ein Fluor-, Chlor- oder Bromatom, eine Methyl- oder eine Methoxygruppe substituierten Phenylrest darstellt,

$X^1$, $X^2$ und $X^3$, die gleich oder verschieden sein können, das Wasserstoffatom, das Fluor-, Chlor- oder Bromatom, eine $C_{1-3}$-Alkyl-, $C_{1-3}$-Alkoxy-, Trifluormethyl-, Hydroxy-, Aminooder Acetylaminogruppe,

A eine Bindung oder den über die -CO-Gruppe mit der $NR^1R^2$-Gruppe der Formel I verknüpften zweiwertigen Rest

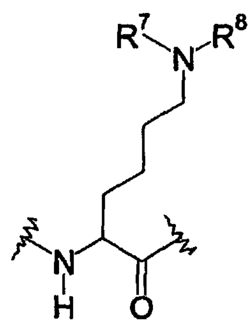

(II),

in dem

$R^7$ und $R^8$ unabhängig voneinander jeweils das Wasserstoffatom oder die Methylgruppe darstellen,

$R^1$ das Wasserstoffatom oder

eine in ω-Stellung gegebenenfalls durch eine Amino-, Methylamino-, Dimethylamino- oder 4-(1-Piperidinyl)-1-piperidinyl-Gruppe substituierte Alkylgruppe mit 1 bis 4 Kohlenstoffatomen,

$R^2$ das Wasserstoffatom, die Methyl- oder Ethylgruppe oder

$R^1$ und $R^2$ zusammen mit dem eingeschlossenen Stickstoffatom einen Rest der allgemeinen Formel

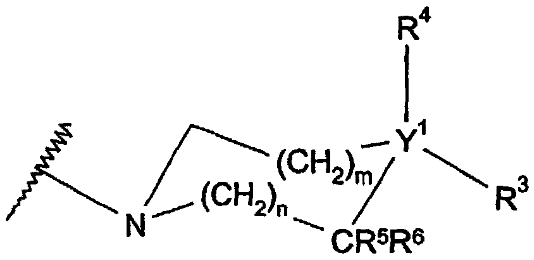

(III),

in der

$Y^1$ das Kohlenstoffatom oder, wenn $R^4$ ein freies Elektronenpaar darstellt, auch das Stickstoffatom,

m die Zahlen 0 oder 1,

n die Zahlen 1 oder 2,

$R^3$ das Wasserstoffatom,

eine Phenyl-, Pyridinyl- oder Diazinylgruppe, die jeweils im Kohlenstoffgerüst durch ein Fluor-, Chlor- oder Bromatom, durch eine Methyl- oder Methoxygruppe substituiert sein können,

eine 5- bis 7-gliedrige Azacycloalkylgruppe, eine 5- bis 7-gliedrige Oxaza- oder Diazacycloalkylgruppe oder eine 7-bis 9-gliedrige Azabicycloalkylgruppe,

wobei die vorstehend genannten mono- und bicyclischen Heterocyclen über ein Stickstoff- oder ein Kohlenstoffatom gebunden sind und

durch eine Alkylgruppe mit 1 bis 3 Kohlenstoffatomen, durch eine $C_{1-4}$-Alkanoyl-, Di-$C_{1-3}$-alkylamino- oder $C_{1-3}$-Alkylsulfonylgruppe substituiert sein können,

$R^4$ ein Wasserstoffatom,

einen Alkylrest mit 1 bis 3 Kohlenstoffatomen, wobei ein unverzweigter Alkylrest in ω-Stellung durch eine Phenyloder Pyridinylgruppe substituiert sein kann,

oder ein freies Elektronenpaar, wenn $Y^1$ ein Stickstoffatom darstellt, und

$R^5$ und $R^6$ jeweils ein Wasserstoffatom darstellen,

bedeuten,

deren Tautomere, deren Diastereomere, deren Enantiomere, deren Gemische und deren Salze.

**2.** Abgewandelte Aminosäureamide der allgemeinen Formel I gemäß Anspruch 1, in denen die Aminosäure-Partialstruktur der Formel

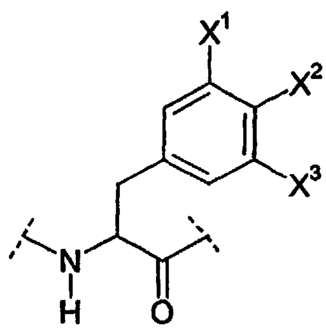

(IV)

D- bzw. (R)-konfiguriert und hinsichtlich der im Rest A gegebenenfalls vorhandenen Aminosäure-Partialstruktur der Formel

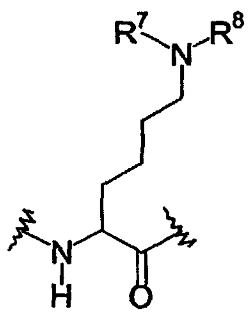

(II)

L- bzw. (S)-konfiguriert ist.

**3.** Abgewandelte Aminosäureamide der allgemeinen Formel I nach mindestens einem der Ansprüche 1 oder 2, in der R die 1-Piperidinylgruppe bedeutet, die in 4-Stellung durch eine 1,3-Dihydro-4-phenyl-2(2H)-oxoimidazol-1-yl-, 1,3-Dihydro-2(2H)-oxobenzimidazol-1-yl-, 2.4(1H,3H)-Dioxochinazolin-3-yl-, 1,3-Dihydro-2(2H)-oxoimidazo[4,5-b]pyridin-3-yl-, 3,4-Dihydro-2(1H)-oxochinazolin-3-yl-, 2,3,4,5-Tetrahydro-2(1H)-oxo-1,3-benzodiazepin-3-yl-, 2(1H)-Oxochinolin-3-yl-, 2,4-Dihydro-5-phenyl-3(3H)-oxo-1,2,4-triazol-2-yl-, 1,3-Dihydro-2(2H)-oxoimidazo[4,5-c]chinolin-3-yl- oder 5,7-Dihydro-6-oxo-dibenzo[d,f][1,3]diazepin-5-yl-Gruppe substituiert ist,

wobei die vorstehend erwähnten bicyclischen Heterocyclen im Kohlenstoffgerüst und/oder an den in diesen Gruppen enthaltenen Phenylgruppen durch Fluor-, Chlor- oder Bromatome, durch Methyl-, Trifluormethyl-, Methoxy-, Hydroxy-, Amino-, Nitro-, Phenyl-, Phenylmethyl-, Carboxy-, Methoxycarbonyl-, Ethoxycarbonyl-, Aminocarbonyl-, Methylaminocarbonyl-, Hydroxyethylaminocarbonyl-, (4-Morpholinyl)carbonyl-, (1-Piperidinyl)carbonyl- oder (4-Methyl-1-piperazinyl)carbonylgruppen mono-, di- oder trisubstituiert sein können,

wobei die Substituenten gleich oder verschieden sein können und eine Mehrfachsubstitution mit den drei letztgenannten Substituenten ausgeschlossen ist,

Y die zweiwertigen Reste

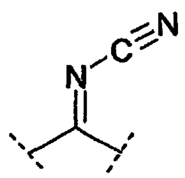

, $-SO_2-$ oder

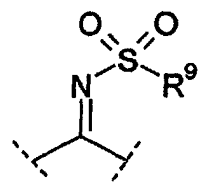

worin $R^9$ die Methylgruppe oder den Phenylrest darstellt,

$X^1$ das Fluor- Chlor- oder Bromatom oder die Methylgruppe,

$X^2$ das Fluor- Chlor- oder Bromatom, die Methyl-, Methoxy-, Hydroxy- oder Aminogruppe,

$X^3$ das Fluor- Chlor- oder Bromatom oder die Methylgruppe,

A eine Bindung oder den über die -CO-Gruppe mit der $NR^1R^2$-Gruppe der Formel I verknüpften zweiwertigen Rest

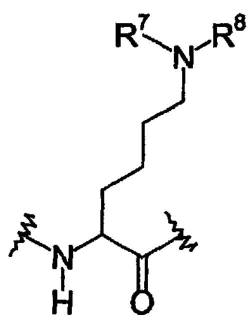

(II),

in dem

$R^7$ und $R^8$ Wasserstoffatome darstellen,

$R^1$ und $R^2$ zusammen mit dem eingeschlossenen Stickstoffatom einen Rest der allgemeinen Formel

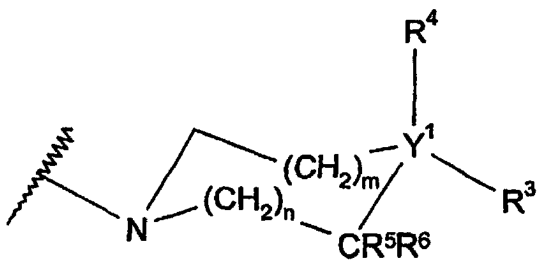

(III),

in der

$Y^1$ das Kohlenstoffatom oder, wenn $R^4$ ein freies Elektronenpaar darstellt, auch das Stickstoffatom,

m die Zahl 1,

n die Zahl 1,

$R^3$ eine Phenyl- oder Pyridinylgruppe, die jeweils im Kohlenstoffgerüst durch ein Fluor-, Chlor- oder Bromatom, durch eine Methyl- oder Methoxygruppe substituiert sein können,

eine 1-Pyrrolidinyl-, 1-Piperidinyl-, 4-(Dimethylamino)-1-piperidinyl-, 4-Piperidinyl- oder 4-Morpholinylgruppe, wobei das Stickstoffatom der 4-Piperidinylgruppe durch eine Alkylgruppe mit jeweils 1 bis 2 Kohlenstoffatomen substituiert sein kann, eine Hexahydro-1H-1-azepinyl-, 4-Methyl-1-piperazinyl- oder 4-Ethyl-1-piperazinylgruppe,

$R^4$ ein Wasserstoffatom, einen Alkylrest mit 1 oder 2 Kohlenstoffatomen oder ein freies Elektronenpaar, wenn $Y^1$ ein Stickstoffatom darstellt, und

$R^5$ und $R^6$ jeweils ein Wasserstoffatom darstellen,

bedeuten,

deren Tautomere, deren Diastereomere, deren Enantiomere, deren Gemische und deren Salze.

**4.** Physiologisch verträgliche Salze der Verbindungen nach mindestens einem der Ansprüche 1 oder 2 mit anorganischen oder organischen Säuren oder Basen.

**5.** Arzneimittel, enthaltend eine Verbindung nach mindestens einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln.

**6.** Verwendung einer Verbindung nach mindestens einem der Ansprüche 1 bis 4 zur Herstellung eines Arzneimittels, das zur akuten und prophylaktischen Behandlung von Kopfschmerzen, zur Behandlung des nicht-insulinabhängigen Diabetes mellitus, von cardiovaskulären Erkrankungen, Erkrankungen der Haut, von entzündlichen Erkrankungen, der allergischen Rhinitis, von Asthma, von Erkrankungen, die mit einer überschießenden Gefäßerweiterung und dadurch bedingter verringerter Gewebedurchblutung einhergehen, der Morphintoleranz oder zur Bekämpfung menopausaler Hitzewallungen geeignet ist.

**7.** Verfahren zur Herstellung eines Arzneimittels gemäß Anspruch 5, **dadurch gekennzeichnet, daß** auf nichtchemischem Wege eine Verbindung nach mindestens einem der Ansprüche 1 bis 4 in einen oder mehrere inerte Trägerstoffe und/oder Verdünnungsmittel eingearbeitet wird.

**8.** Verfahren zur Herstellung der Verbindungen der allgemeinen Formel I nach mindestens einem der Ansprüche 1 oder 2, **dadurch gekennzeichnet, daß**

a) zur Herstellung von Verbindungen der allgemeinen Formel I, in der Y einen der zweiwertigen Iminomethylreste

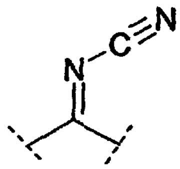

oder

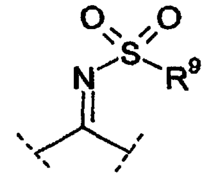

bedeutet, wobei $R^9$ wie in den Ansprüchen 1 bis 4 definiert ist,
eine Verbindung der allgemeinen Formel

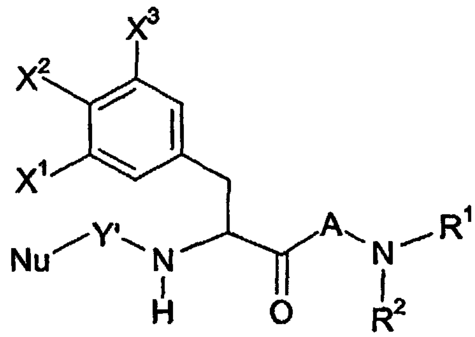

(V),

in der A, $R^1$, $R^2$, $X^1$, $X^2$ und $X^3$ wie in den Ansprüchen 1 bis 4 definiert sind, Y' einen der beiden oben angegebenen Iminomethylreste darstellt und Nu eine Austrittsgruppe ist,
mit einem sekundären Aminen der allgemeinen Formel

R-H (VI),

in der R wie in den Ansprüchen 1 bis 4 definiert ist, umgesetzt wird oder

b) zur Herstellung von Verbindungen der allgemeinen Formel I, in der Y den zweiwertigen Rest $-SO_2-$ bedeutet, eine Verbindung der allgemeinen Formel

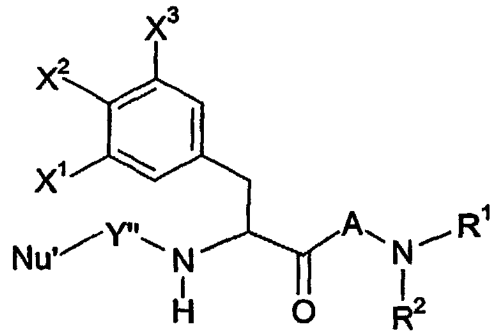

(VII),

in der A, $R^1$, $R^2$, $X^1$, $X^2$ und $X^3$ wie in den Ansprüchen 1 bis 4 definiert sind, Y" die $SO_2$-Gruppe bedeutet und Nu' eine Austrittsgruppe ist,
mit einem sekundären Amin der allgemeinen Formel

R-H (VI),

in der R wie in den Ansprüchen 1 bis 4 definiert ist, umgesetzt wird und
erforderlichenfalls ein bei den vorstehend beschriebenen Umsetzungen verwendeter Schutzrest wieder abgespalten wird und/oder
gegebebenenfalls verwendete Präcursorfunktionen in einer so erhaltenen Verbindung abgewandelt werden und/oder
gewünschtenfalls eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze übergeführt wird.

**9.** Verwendung der Verbindungen der allgemeinen Formel I nach mindestens einem der Ansprüche 1 oder 2 zur Erzeugung und Reinigung von Antikörpern.

**10.** Verwendung markierter Verbindungen der allgemeinen Formel I nach mindestens einem der Ansprüche 1 oder 2 in RIA- und ELISA-Assays und als diagnostische oder analytische Hilfsmittel in der Neurotransmitter-Forschung.

## Claims

**1.** Modified amino acid amides of general formula

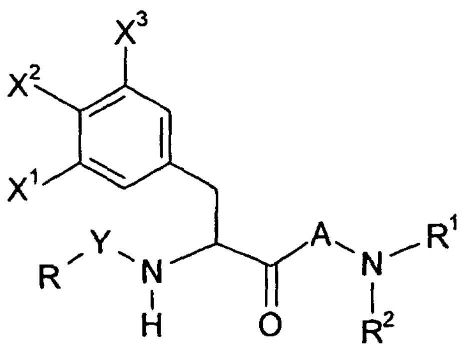

(I),

wherein
R denotes the 1-piperidinyl group, which is substituted in the 4 position by a mono- or di-unsaturated 5- to 7-membered aza, diaza or triaza heterocyclic group, bound via a nitrogen atom, which contains one or two carbonyl groups linked to a nitrogen atom,

whilst the abovementioned heterocyclic groups may be substituted at a carbon atom by a phenyl group,

an olefinic double bond of one of the abovementioned unsaturated heterocyclic groups may be condensed with a benzene, pyridine or quinoline ring or two olefinic double bonds in one of the abovementioned unsaturated heterocyclic groups may be benzocondensed,

and whilst the abovementioned condensed heterocyclic groups may be mono-, di- or trisubstituted in the carbon skeleton and/or at the phenyl groups contained in these groups by fluorine, chlorine or bromine atoms, by $C_{1-3}$ -alkyl, trifluoromethyl, $C_{1-3}$-alkoxy, hydroxy, amino, nitro, phenyl, carboxy, methoxycarbonyl, ethoxycarbonyl, aminocarbonyl, methylaminocarbonyl, hydroxyethylaminocarbonyl, (4-morpholinyl)carbonyl, (1-piperidinyl)carbonyl or (4-methyl-1-piperazinyl)carbonyl groups,

whilst the substituents may be identical or different and multiple substitution with the three latter substituents is excluded,

Y denotes the divalent groups

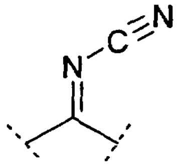

, $-SO_2-$ or

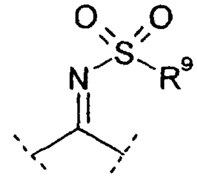

wherein $R^9$ denotes a $C_{1-3}$-alkyl group or a phenyl group optionally substituted by a fluorine, chlorine or bromine atom, or a methyl or methoxy group,
$X^1$, $X^2$ and $X^3$, which may be identical or different, denote the hydrogen atom, the fluorine, chlorine or bromine atom, a $C_{1-3}$-alkyl, $C_{1-3}$-alkoxy, trifluoromethyl, hydroxy, amino or acetylamino group,
A denotes a bond or the divalent group

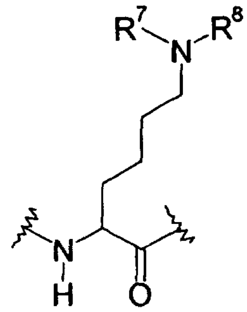

(II),

linked to the $NR^1R^2$ group of formula I via the -CO group, wherein
$R^7$ and $R^8$ independently of each other in each case denote the hydrogen atom or the methyl group,
$R^1$ denotes the hydrogen atom or
an alkyl group with 1 to 4 carbon atoms optionally substituted in the ω position by an amino, methylamino, dimethylamino or 4-(1-piperidinyl)-1-piperidinyl group,
$R^2$ denotes the hydrogen atom, the methyl or ethyl group or
$R^1$ and $R^2$ together with the enclosed nitrogen atom denote a group of general formula

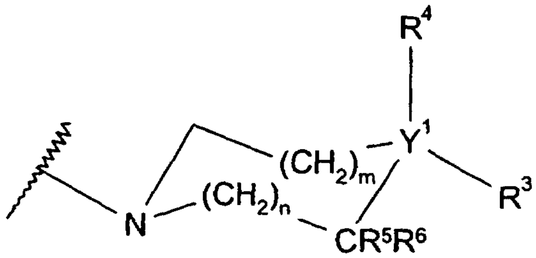

(III),

wherein
$Y^1$ denotes the carbon atom or, if $R^4$ denotes a free pair of electrons, it may also represent the nitrogen atom,
m denotes the numbers 0 or 1,
n denotes the numbers 1 or 2,
$R^3$ denotes the hydrogen atom,
a phenyl, pyridinyl or diazinyl group, each of which may be substituted in the carbon skeleton by a fluorine, chlorine or bromine atom or by a methyl or methoxy group,
a 5- to 7-membered azacycloalkyl group, a 5- to 7-membered oxaza or diazacycloalkyl group or a 7- to 9-membered azabicycloalkyl group,
whilst the abovementioned mono- and bicyclic heterocyclic groups are bound via a nitrogen or carbon atom and
may be substituted by an alkyl group with 1 to 3 carbon atoms, by a $C_{1-4}$-alkanoyl, di-$C_{1-3}$ -alkylamino or $C_{1-3}$ -alkylsulphonyl group,
$R^4$ denotes a hydrogen atom,
an alkyl group with 1 to 3 carbon atoms, whilst an unbranched alkyl group may be substituted in the ω position by a phenyl or pyridinyl group,
or a free pair of electrons, if $Y^1$ denotes a nitrogen atom, and
$R^5$ and $R^6$ in each case denote a hydrogen atom,
the tautomers, the diastereomers, the enantiomers, the mixtures and the salts thereof.

**2.** Modified amino acid amides of general formula I according to claim 1, wherein the amino acid partial structure of formula

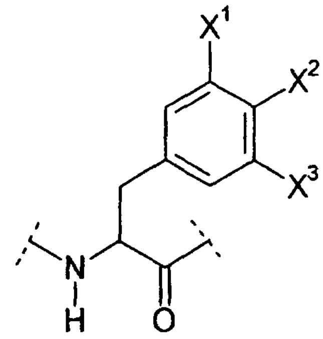

(IV)

is in the D- or (R)-configuration and the amino acid partial structure of formula

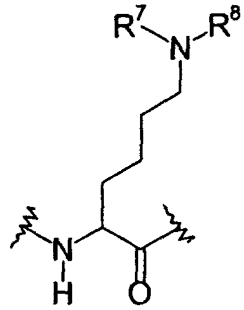

(II)

which may optionally be present in the group A is in the L- or (S)-configuration.

**3.** Modified amino acid amides of general formula I according to at least one of claims 1 or 2, wherein R denotes the 1-piperidinyl group which is substituted in the 4 position by a 1,3-dihydro-4-phenyl-2(2H)-oxoimidazol-l-yl, 1,3-dihydro-2(2H)-oxobenzimidazol-1-yl, 2,4(1H,3H)-dioxoquinazolin-3-yl, 1,3-dihydro-2(2H)-oxoimidazo[4,5-b]pyridin-3-yl, 3,4-dihydro-2(1H)-oxoquinazolin-3-yl, 2,3,4,5-tetrahydro-2(1H)-oxo-1,3-benzodiazepin-3-yl, 2(1H)-oxoquinolin-3-yl, 2,4-dihydro-5-phenyl-3(3H)-oxo-1,2,4-triazol-2-yl, 1,3-dihydro-2(2H)-oxoimidazo[4,5-c]quinolin-3-yl or 5,7-dihydro-6-oxo-dibenzo[d,f][1,3]diazepin-5-yl group,

whilst the abovementioned bicyclic heterocyclic groups may be mono-, di- or trisubstituted in the carbon skeleton and/or at the phenyl groups contained in these groups by fluorine, chlorine or bromine atoms, by methyl, trifluoromethyl, methoxy, hydroxy, amino, nitro, phenyl, phenylmethyl, carboxy, methoxycarbonyl, ethoxycarbonyl, aminocarbonyl, methylaminocarbonyl, hydroxyethylaminocarbonyl, (4-morpholinyl)carbonyl, (1-piperidinyl) carbonyl or (4-methyl-l-piperazinyl)carbonyl groups,

whilst the substituents may be identical or different and multiple substitution with the three latter substituents is ruled out,

Y denotes the divalent groups

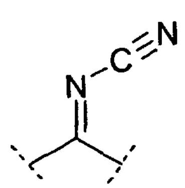

, $-SO_2-$ or

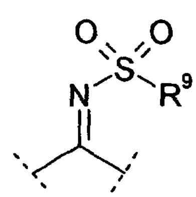

wherein $R^9$ denotes the methyl group or the phenyl group,

$X^1$ denotes the fluorine, chlorine or bromine atom or the methyl group,

$X^2$ denotes the fluorine, chlorine or bromine atom, the methyl, methoxy, hydroxy or amino group,

$X^3$ denotes the fluorine, chlorine or bromine atom or the methyl group,
A denotes a bond or the divalent group

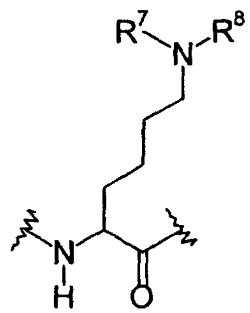

(II),

linked to the $NR^1R^2$ group of formula I via the -CO group, wherein
$R^7$ and $R^8$ denote hydrogen atoms,
$R^1$ and $R^2$ together with the enclosed nitrogen atom denote a group of general formula

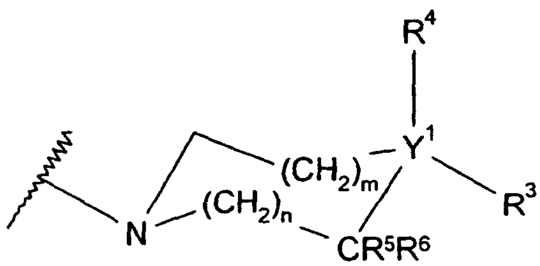

(III),

wherein
$Y^1$ denotes the carbon atom or, if $R^4$ denotes a free pair of electrons, it may also represent the nitrogen atom,
m denotes the number 1,
n denotes the number 1,
$R^3$ denotes a phenyl or pyridinyl group, each of which . may be substituted in the carbon skeleton by a fluorine, chlorine or bromine atom, or by a methyl or methoxy group,
a 1-pyrrolidinyl, 1-piperidinyl, 4-(dimethylamino)-1-piperidinyl, 4-piperidinyl or 4-morpholinyl group, whilst the nitrogen atom of the 4-piperidinyl group may be substituted by an alkyl group with 1 to 2 carbon atoms in each case, a hexahydro-1H-1-azepinyl, 4-methyl-1-piperazinyl or 4-ethyl-1-piperazinyl group,
$R^4$ denotes a hydrogen atom, an alkyl group with 1 or 2 carbon atoms or a free pair of electrons, if $Y^1$ denotes a nitrogen atom, and
$R^5$ and $R^6$ in each case denote a hydrogen atom,
the tautomers, the diastereomers, the enantiomers, the mixtures and the salts thereof.

4. Physiologically acceptable salts of the compounds according to at least one of claims 1 or 2 with inorganic or organic acids or bases.

5. Pharmaceutical compositions containing a compound according to at least one of claims 1 to 3 or a physiologically acceptable salt thereof according to claim 4 optionally together with one or more inert carriers and/or diluents.

6. Use of a compound according to at least one of claims 1 to 4 for preparing a pharmaceutical composition which is suitable for the acute and prophylactic treatment of headaches, for treating non-insulin-dependent diabetes mellitus, cardiovascular diseases, skin diseases, inflammatory diseases, allergic rhinitis, asthma, diseases **characterised by** excessive vasodilatation and a consequent reduction in bloodflow through the tissues, or morphine tolerance, or for treating menopausal hot flushes.

7. Process for preparing a pharmaceutical composition according to claim 5, **characterised in that** a compound according to at least one of claims 1 to 4 is incorporated in one or more inert carriers and/or diluents by a non-chemical method.

**8.** Process for preparing the compounds of general formula I according to at least one of claims 1 or 2, **characterised in that**

a) in order to prepare compounds of general formula I, wherein Y denotes one of the divalent iminomethyl groups

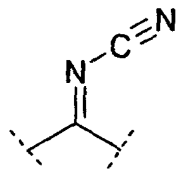

or

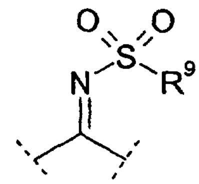

wherein $R^9$ is defined as in claims 1 to 4,
a compound of general formula

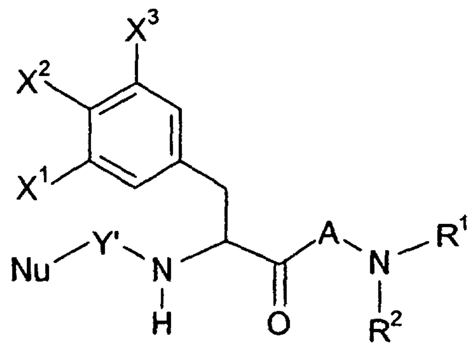

(V),

wherein A, $R^1$, $R^2$, $X^1$, $X^2$ and $X^3$ are defined as in claims 1 to 4, Y' denotes one of the two abovementioned iminomethyl groups and Nu is a leaving group,
is reacted with a secondary amine of general formula

$$R\text{-}H \qquad (VI),$$

wherein R is defined as in claims 1 to 4, or
b) in order to prepare compounds of general formula I wherein Y denotes the divalent group $-SO_2-$,
a compound of general formula

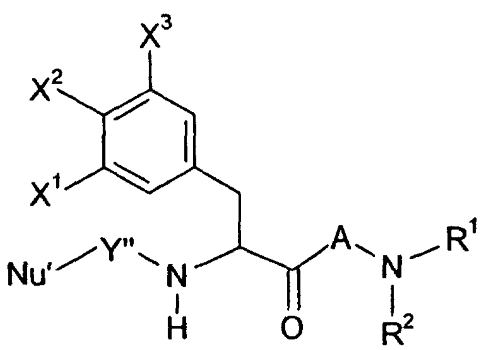

(VII),

wherein A, $R^1$, $R^2$, $X^1$, $X^2$ and $X^3$ are defined as in claims 1 to 4, Y'' denotes the $SO_2$ group and Nu' is a leaving group,

is reacted with a secondary amine of general formula

R-H (VI),

wherein R is defined as in claims 1 to 4, and
if necessary any protecting group used during the reactions described above is cleaved and/or
any precursor functions used in a compound thus obtained are modified and/or
if desired a compound of general formula I thus obtained is resolved into its stereoisomers and/or
a compound of general formula I thus obtained is converted into the salts thereof, particularly for pharmaceutical use into the physiologically acceptable salts thereof.

**9.** Use of the compounds of general formula I according to at least one of claims 1 or 2 for the production and purification of antibodies.

**10.** Use of labelled compounds of general formula I according to at least one of claims 1 or 2 in RIA and ELISA assays and as diagnostic or analytical aids in neurotransmitter research.

## Revendications

**1.** Amide d'acide aminé modifié de la formule générale :

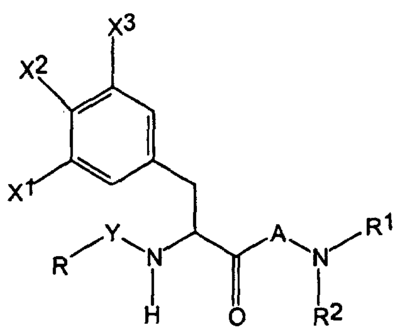

(I)

dans laquelle

R représente le groupe 1-pipéridinyle, qui est substitué en position 4 par un aza-, diaza- ou triazahétérocycle ayant 5 à 7 membres, une ou deux fois insaturé, relié par un atome d'azote, qui contient un ou deux groupes carbonyle reliés à un atome d'azote,

dans laquelle les hétérocycles indiqués ci-dessus peuvent être substitués sur un atome de carbone par un groupe phényle, .

dans laquelle une double liaison oléfinique d'un des hétérocyles insaturés indiqués ci-dessus, peut être condensée avec un cycle benzène, pyridine ou quinoléine ou deux doubles liaisons oléfiniques d'un des hétérocycles insaturés indiqués ci-dessus, peuvent être benzocondensées,

et dans laquelle les hétérocycles condensés indiqués ci-dessus peuvent être mono-, di- ou trisubstitués dans le squelette carboné et/ou sur le groupe phényle présent dans ces groupes, par l'atome de fluor, de chlore ou de brome, par un groupe alkyle en $C_{1-3}$, trifluorométhyle, alcoxy en $C_{1-3}$, hydroxyle, amino, nitro, phényle, carboxyle, méthoxycarbonyle, éthoxycarbonyle, aminocarbonyle, méthylaminocarbonyle, hydroxyéthylaminocarbonyle, (4-morpholinyl)carbonyle, (1-pipéridinyl)-carbonyle ou (4-méthyl-1-pipérazinyl)carbonyle,

dans laquelle les substituants peuvent être identiques ou différents et une multisubstitution avec les trois derniers substituants est exclue ;

Y représente le reste bivalent :

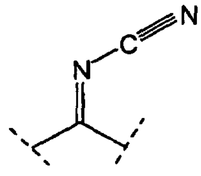 $-SO_2-$ ou 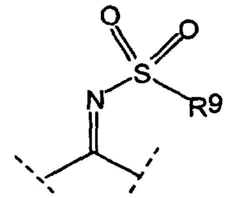

dans lequel $R^9$ représente un reste alkyle en $C_{1-3}$ ou un reste phényle le cas échéant substitué par l'atome de fluor, de chlore ou de brome, le groupe méthyle ou méthoxy ;
$X^1$, $X^2$ et $X^3$, qui peuvent être identiques ou différents, représentent l'atome d'hydrogène, de fluor, de chlore ou de brome, un groupe alkyle en $C_{1-3}$, alcoxy en $C_{1-3}$, trifluorométhyle, hydroxyle, amino ou acétylamino ;
A représente une liaison ou le reste bivalent relié par le groupe CO avec le groupe $NR^1R^2$ de la formule I

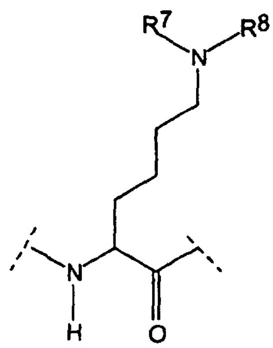 (II)

dans laquelle
$R^7$ et $R^8$ représentent indépendamment l'un de l'autre, l'atome d'hydrogène ou le groupe méthyle ;
$R^1$ représente l'atome d'hydrogène, ou
un groupe alkyle ayant 1 à 4 atomes de carbone, le cas échéant substitué en position ω par le groupe amino, méthylamino, diméthylamino ou 4-(1-pipéridinyl)-1-pipéridinyle ;
$R^2$ représente l'atome d'hydrogène, le groupe méthyle
ou éthyle, ou
$R^1$ et $R^2$ avec l'atome d'azote, représentent un reste de la formule générale :

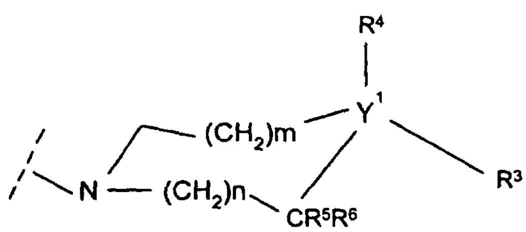 (III)

dans laquelle
$Y^1$ représente l'atome de carbone, ou lorsque $R^4$ représente une paire d'électrons libre, également l'atome d'azote ;
m représente le nombre 0 ou 1 ;
n représente le nombre 1 ou 2 ;
$R^3$ représente l'atome d'hydrogène,
un groupe phényle, pyridinyle ou diazinyle, qui peut être substitué chaque fois sur le squelette carboné, par l'atome de fluor, de chlore ou de brome, par le groupe méthyle ou méthoxy,

un groupe azacycloalkyle ayant 5 à 7 membres, un groupe oxaza- ou diazacycloalkyle ayant 5 à 7 membres, ou un groupe azabicycloalkyle ayant 7 à 9 membres,

où les hétérocycles mono- et bicycliques indiqués ci-dessus, sont reliés par un atome d'azote ou de carbone, et

et peuvent être substitués par un groupe alkyle ayant 1 à 3 atomes de carbone, par un groupe alcanoyle en $C_{1-4}$, di (alkyl en $C_{1-3}$)amino ou (alkyl en $C_{1-3}$) sulfonyle,

$R^4$ représente l'atome d'hydrogène,

un groupe alkyle ayant 1 à 3 atomes de carbone, où un reste alkyle non ramifié peut être substitué en position ω par un groupe phényle ou pyridinyle,

ou une paire d'électrons libres, lorsque $Y^1$ représente un atome d'azote, et

$R^5$ et $R^6$ représentent chaque fois, l'atome d'hydrogène,

leurs tautomères, leurs diastéréoisomères, leurs énantiomères, leurs mélanges et leurs sels.

**2.** Amide d'acide aminé modifié de la formule générale I selon la revendication 1, dans lequel la structure partielle d'acide aminé de la formule :

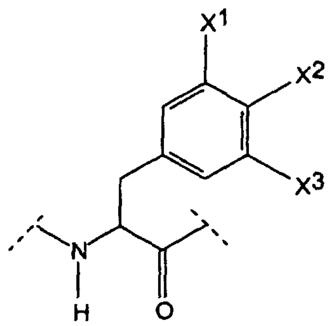

(IV)

a la configuration D ou R, et la structure partielle d'acide aminé présente le cas échéant dans le reste A, de formule :

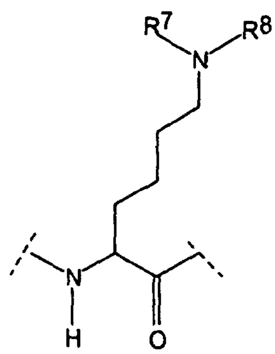

(II)

a la configuration L ou S.

**3.** Amide d'acide aminé modifié de la formule générale I selon au moins l'une des revendications 1 1 ou 2, dans lequel

R représente le groupe 1-pipéridinyle, qui est substitué en position 4, par un groupe 1,3-dihydro-4-phényl-2(2H)-oxoimidazol-1-yle, 1,3-dihydro-2(2H)-oxobenzimidazol-1-yle, 2,4(1H,3H)-dioxoquinazolin-3-yle, 1,3-dihydro-2(2H)-oxoimidazo[4,5-b]pyridin-3-yle, 3,4-dihydro-2(1H)- oxoquinazolin-3-yle, 2,3,4,5-tétrahydro-2 (1H)-oxo-1,3-benzodiazépin-3-yle, 2(1H)-oxoquinolin-3-yle, 2,4-dihydro-5-phényl-3(3H)-oxo-1,2,4-triazol-2-yle, 1,3-dihydro-2 (2H) -oxoimidazo[4,5-c]quinolin-3-yle, ou 5,7-dihydro-6-oxodibenzo[d, f][1,3]diazépin-5-yle,

dans lequel les hétérocycles bicycliques indiqués ci-dessus peuvent être mono-, di- ou trisubstitués dans le squelette carboné et/ou sur le groupe phényle présent dans ces groupes, par l'atome de fluor, de chlore ou de brome, le groupe méthyle, trifluorométhyle, méthoxy, hydroxyle, amino, nitro, phényle, phénylméthyle, carboxyle, méthoxycarbonyle, éthoxycarbonyle, aminocarbonyle, méthylaminocarnonyle, hydroxyéthylaminocarbonyle, (4-morpholinyl)carbonyle, (1-pipéridinyl)carbonyle ou (4-méthyl-1-pipérazinyl)-carbonyle,

dans lequel les substituants peuvent être identiques ou différents et une multisubstitution avec les trois derniers substituants cités est exclue ;
Y représente le reste bivalent :

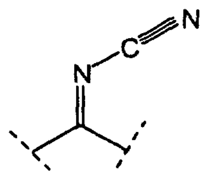

-$SO_2$-

ou

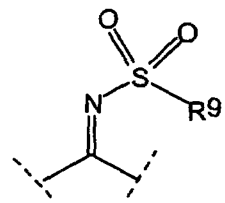

dans lequel $R^9$ représente le groupe méthyle ou le groupe phényle ;
$X^1$ représente l'atome de fluor, de chlore ou de brome, ou le groupe méthyle ;
$X^2$ représente l'atome de fluor, de chlore ou de brome, ou le groupe méthyle, méthoxy, hydroxyle ou amino ;
$X^3$ représente l'atome de fluor, de chlore ou de brome, ou le groupe méthyle ;
A représente une liaison ou le groupe bivalent relié par le groupe CO avec le groupe $NR^1R^2$ de la formule I

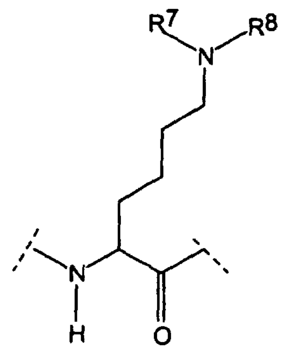

(II)

dans laquelle
$R^7$ et $R^8$ représentent l'atome d'hydrogène ;
$R^1$ et $R^2$ avec l'atome d'azote, représentent un reste de la formule générale :

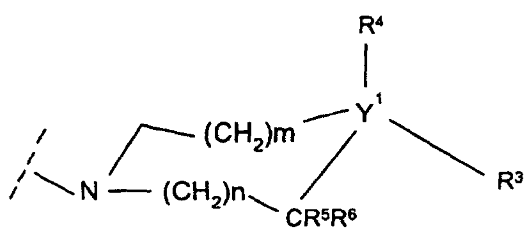

(III)

dans laquelle
$Y^1$ représente l'atome de carbone, ou lorsque $R^4$ représente une paire d'électrons libres, également l'atome d'azote ;
m représente le nombre 1 ;
n représente le nombre 1 ;
$R^3$ représente un groupe phényle ou pyridinyle, qui peut être substitué chaque fois sur le squelette carboné, par l'atome de fluor, de chlore ou de brome, par le groupe méthyle ou méthoxy,
un groupe 1-pyrrolidinyle, 1-pipéridinyle, 4-(diméthylamino)-1-pipéridinyle, 4-pipéridinyle ou 4-morpholinyle, dans lequel l'atome d'azote du groupe 4-pipéridinyle peut être substitué par un groupe alkyle ayant 1 à 2 atomes de carbone, un groupe hexahydro-1H-1-azépinyle, 4-méthyl-1-pipérazinyle ou 4-éthyl-1-pipérazinyle ;

$R^4$ représente l'atome d'hydrogène, un groupe alkyle ayant 1 ou 2 atomes de carbone, ou une paire d'électrons libres, lorsque $Y^1$ représente un atome d'azote, et

$R^5$ et $R^6$ représentent chaque fois, l'atome d'hydrogène,

leurs tautomères, leurs diastéréoisomères, leurs énantiomères, leurs mélanges et leurs sels.

**4.** Sels compatibles d'un point de vue physiologique, des composés selon au moins l'une des revendications 1 ou 2, avec des acides ou bases inorganiques ou organiques.

**5.** Agent pharmaceutique contenant un composé selon au moins l'une des revendications 1 à 3 ou un sel compatible d'un point de vue physiologique selon la revendication 4, et le cas échéant, un ou plusieurs supports et/ou agents de dilution inertes.

**6.** Utilisation d'un composé selon au moins l'une des revendications 1 à 4, pour la préparation d'un agent pharmaceutique, qui est approprié pour le traitement aigu ou prophylactique des maux de tête, pour le traitement du diabète non insulino-dépendant, des maladies cardiovasculaires, de maladies de la peau, de maladies infectieuses, de la rhinite allergique, de l'asthme, de maladies qui se développent avec un élargissement excessif des vaisseaux et ainsi, une irrigation diminuée des tissus, de l'accoutumance à la morphine ou pour lutter contre les bouffées de chaleur de la ménopause.

**7.** Procédé de préparation d'un agent pharmaceutique selon la revendication 5, **caractérisé en ce que** l'on incorpore par une voie non chimique, un composé selon au moins l'une des revendications 1 à 4, dans un ou plusieurs supports et/ou agents de dilution inertes.

**8.** Procédé de préparation d'un composé de la formule générale I selon au moins l'une des revendications 1 ou 2, **caractérisé en ce que**

a) pour préparer des composés de la formule générale I, dans laquelle Y représente l'un des restes iminométhyle bivalents :

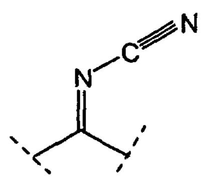

ou

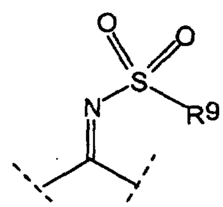

dans lequel $R^9$ est défini comme aux revendications 1 à 4,

on fait réagir un composé de la formule générale :

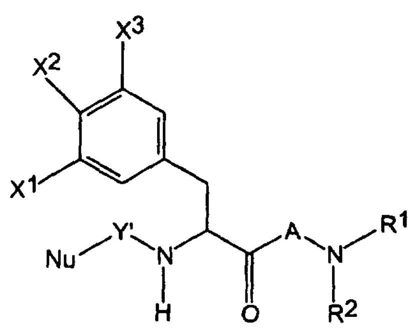

(IV)

dans laquelle A, $R^1$, $R^2$, $X^1$, $X^2$ et $X^3$ sont définis comme aux revendications 1 à 4, Y' représente l'un des deux restes iminométhyle représentés ci-dessus et Nu est un groupe partant,

avec une amine secondaire de la formule générale :

R - H (VI)

dans laquelle R est défini comme aux revendications 1 à 4,
ou

b) pour préparer des composés de la formule générale I, dans laquelle Y représente le reste bivalent $SO_2$, on fait réagir un composé de la formule générale :

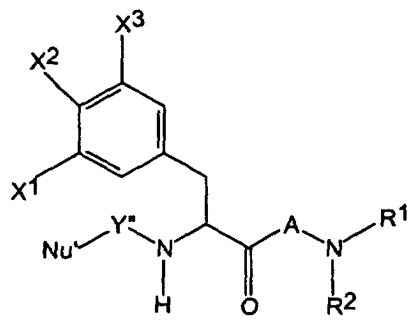

(VII)

dans laquelle A, $R^1$, $R^2$, $X^1$, $X^2$ et $X^3$ sont définis comme aux revendications 1 à 4, Y" représente le groupe $SO_2$ et Nu' est un groupe partant,
avec une amine secondaire de la formule générale :

R - H (VI)

dans laquelle R est défini comme aux revendications 1 à 4,
et si nécessaire, un reste protecteur utilisé lors des réactions décrites ci-dessus, est clivé et/ou
des fonctions précurseurs le cas échéant utilisées dans un composé ainsi obtenu sont modifiées et/ou
si désiré, un composé ainsi obtenu de la formule générale I est séparé en ses stéréoisomères et/ou
un composé ainsi obtenu de la formule générale I est converti en son sel, en particulier pour l'utilisation pharmaceutique, en son sel compatible d'un point de vue physiologique.

**9.** Utilisation des composés de la formule générale I selon au moins l'une des revendications 1 ou 2, pour produire et purifier des anticorps.

**10.** Utilisation de composés marqués de la formule générale I selon au moins l'une des revendications 1 ou 2, dans des dosages RIA et ELISA et comme auxiliaire diagnostique ou analytique dans la recherche sur les neurotransmetteurs.